# EUROPÄISCHE PATENTANMELDUNG

(11) **EP 0 757 039 A1**
(43) Veröffentlichungstag der Anmeldung: **05.02.1997**
(21) Anmeldenummer: 96112347.8
(22) Anmeldetag: 31.07.1996
(51) Int. Cl.: C07D 215/54, A61K 31/47, C07D 405/04, C07D 405/12, C07D 417/04, C07D 417/14

(54) **Endothelin-Rezeptor-Antagonisten**

(30) Priorität: 02.08.1995 DE 19528418
(71) Anmelder: MERCK PATENT GmbH, 64293 Darmstadt (DE)
(72) Erfinder: Mederski, Werner, Dr., 64390 Erzhausen (DE); Osswald, Mathias, Dr., 64673 Zwingenberg (DE); Dorsch, Dieter, Dr., 64372 Ober-Ramstadt (DE); Wilm, Claudia, Dr., 64367 Mühltal (DE); Schmitges, Claus J., Dr., 64823 Gross-Umstadt (DE); Christadler, Maria, 63322 Rödermark (DE)

(57) **Zusammenfassung**

Neue Verbindungen der Formel I 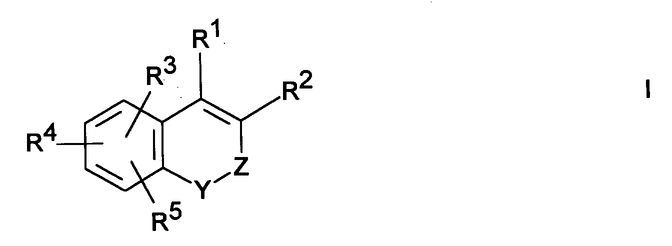 worin
-Y-Z-, R¹, R², R³, R⁴ und R⁵ die in Patentanspruch 1 angegebene Bedeutung haben, sowie deren Salze
zeigen Endothelinrezeptor-antagonistische Eigenschaften.

## Beschreibung

Die Erfindung betrifft Verbindungen der Formel I 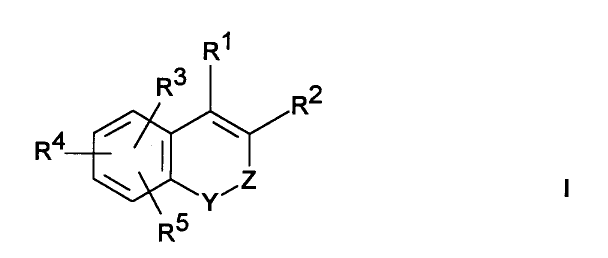 worin
- -Y-Z-: -NR⁷-CO-, -N=C(OR⁷)- oder -N=CR⁸-,
- R¹: Ar,
- R²: COOR⁶, (CH₂)ₙCOOR⁶, CN, 1H-Tetrazol-5-yl oder CONHSO₂Ar,
- R³, R⁴, R⁵: jeweils unabhängig voneinander R⁶, OR⁶, S(O)ₘR⁶, Hal, NO₂, NR⁶R⁶', NHCOR⁶, NHSO₂R⁶, OCOR⁶, COR⁶, COOR⁶ oder CN, wobei R³ und R⁴ zusammen auch eine O(CH₂)ₙO-Gruppe darstellen können,
- R⁶, R^{6'}: jeweils unabhängig voneinander H, Alkyl mit 1 bis 6 C-Atomen, Benzyl oder Phenyl,
- R⁷: (CH₂)ₙAr,
- R⁸: Ar oder OAr,
- Ar: unsubstituiertes oder ein-, zwei- oder dreifach durch R⁹, R¹⁰ oder R¹¹ substituiertes Phenyl oder
unsubstituiertes Naphthyl oder
eine unsubstituierte oder im Phenylteil ein- oder zweifach durch R⁹ oder R¹⁰ substituierte 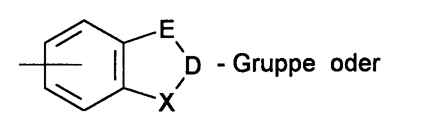 eine unsubstituierte oder im Cyclohexadienylteil ein- oder zweifach durch R⁹ oder R¹⁰ substituierte 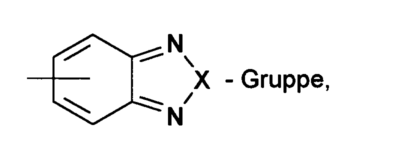
- R⁹, R¹⁰, R¹¹: jeweils unabhängig voneinander R⁶, OR⁶, Hal, CF₃, OCF₃, OCHF₂, OCH₂F, NO₂, NR⁶R^{6'}, NHCOR⁶, CN, NHSO₂R⁶, COOR⁶, COR⁶, CONHSO₂Ar, O(CH₂)ₙR², O(CH₂)ₙOR⁶ oder S(O)ₘR⁶,
- E: CH₂, S oder O,
- D: Carbonyl oder [C(R⁶R^{6'})]ₙ,
- X: O oder S,
- Hal: F, Cl, Br oder I,
- m: 0, 1 oder 2,
- n: 1, 2 oder 3 bedeuten,
sowie ihre Salze.

Ähnliche Verbindungen mit Indan- und Indengrundgerüsten sind aus WO 93/08799, solche mit Indolsystemen sind aus WO 94/14434, Pyrimidinderivate sind aus EP 0 526 708 A1 und Phenyl- und Naphthylverbindungen sind aus EP 0 617 001 A1 bekannt.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze bei guter Verträglichkeit sehr wertvolle pharmakologische Eigenschaften besitzen. Insbesondere zeigen sie Endothelinrezeptor-antagonistische Eigenschaften und können daher zur Behandlung von Krankheiten wie Hypertonie, Herzinsuffizienz, koronare Herzerkrankung, renale, cerebrale und myocardiale Ischämie, Niereninsuffizienz, Hirninfarkt, subarachnoidale Hämorrhagie, Arteriosklerose, pulmonaler Hochdruck, Entzündungen, Asthma, Prostatahyperplasie, endotoxischer Schock und bei Komplikationen nach der Verabreichung von Substanzen wie z.B. Cyclosporin, sowie anderen, mit Endothelin-Aktivitäten assoziierten Krankheiten eingesetzt werden.

Die Verbindungen zeigen u.a. eine hohe Affinität zu den Endothelin-Subrezeptoren ET_{A} und ET_{B}. Diese Wirkungen können nach üblichen in vitro- oder in vivo-Methoden ermittelt werden, wie z.B. beschrieben von P.D. Stein et al., J. Med. Chem 37, 1994, 329-331 und E. Ohlstein et al., Proc. Natl. Acad. Sci. USA 91, 1994, 8052-8056.

Eine geeignete Methode zur Bestimmung der blutdrucksenkenden Wirkung wird z. B. beschrieben von M.K. Bazil et al., J. Cardiovasc. Pharmacol 22, 1993, 897-905 und J. Lange et al., Lab Animal 20, 1991, Appl. Note 1016.

Die Verbindungen der Formel I können als Arzneimittelwirkstoffe in der Human- und Veterinärmedizin eingesetzt werden, insbesondere zur Prophylaxe und/oder Therapie von Herz-, Kreislauf- und Gefäßkrankheiten, vor allem von Hypertonie und Herzinsuffizienz.

Gegenstand der Erfindung sind die Verbindungen der Formel I und ihre Salze sowie ein Verfahren zur Herstellung dieser Verbindungen sowie ihrer Salze, dadurch gekennzeichnet, daß man
(a) zur Herstellung einer Verbindung der Formel I
   worin
   - -Y-Z-: -NR⁷-CO- oder -N=C(OR⁷)- bedeutet,
   eine Verbindung der Formel II 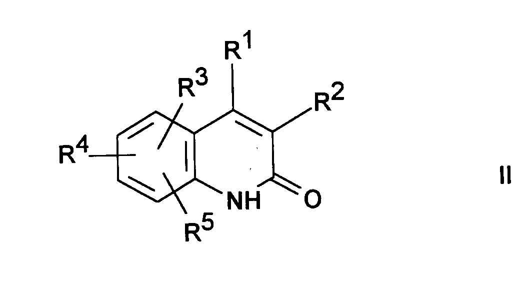 worin
   R¹, R², R³, R⁴ und R⁵ die in Anspruch 1 angegebenen Bedeutungen haben,
   mit einer Verbindung der Formel III

   R⁷-Q III

   worin
   - Q: Cl, Br, I oder eine freie oder reaktionsfähig funktionell abgewandelte OH-Gruppe bedeutet und
   - R⁷: die in Anspruch 1 angegebene Bedeutung hat,
   umsetzt,
   oder daß man
(b) zur Herstellung einer Verbindung der Formel I,
   worin
   - -Y-Z-: -N=C(Ar)- bedeutet,
   eine Verbindung der Formel IV 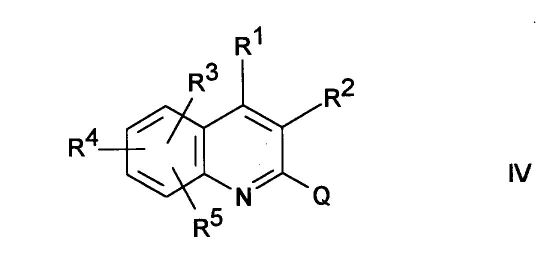 worin
   - Q: Cl, Br oder I bedeutet und
   R¹, R², R³, R⁴ und R⁵ die in Anspruch 1 angegebenen Bedeutungen haben,
   mit einer Borverbindung der Formel V

   Ar-BLL' V

   worin
   - L, L': jeweils unabhängig voneinander OH, OCH₃, OC₂H₅ oder OC₃H₇ bedeutet, und
   Ar die in Anspruch 1 angegebene Bedeutung hat,
   umsetzt,
   oder daß man
c) zur Herstellung einer Verbindung der Formel I,
   worin
   - -Y-Z-: -N=C(OAr)- bedeutet,
   eine Verbindung der Formel IV
   worin
   - Q: Cl, Br, I oder eine reaktionsfähig funktionell abgewandelte OH-Gruppe bedeutet,
   mit einer Verbindung der Formel VI

   Ar-OH VI

   umsetzt,
   und/oder daß man in einer Verbindung der Formel I einen oder mehrere Rest(e) R¹ ,R², R³, R⁴ und/oder R⁵ in einen oder mehrere Rest(e) R¹ ,R², R³, R⁴ und/oder R⁵ umwandelt,
   indem man beispielsweise
   i) eine Nitrogruppe zu einer Aminogruppe reduziert,
   ii) eine Estergruppe zu einer Carboxygruppe hydrolysiert,
   iii) eine Aminogruppe durch reduktive Aminierung in ein alkyliertes Amin umwandelt,
   iv) eine Carboxygruppe in eine Sulfonamidocarbonylgruppe umwandelt
   und/oder eine Base oder Säure der Formel I in eines ihrer Salze umwandelt.

Für alle Reste, die mehrfach auftreten, wie z. B. R⁶ und Ar, gilt, daß deren Bedeutungen unabhängig voneinander sind.

In den vorstehenden Formeln hat Alkyl 1 bis 6, vorzugsweise 1, 2, 3 oder 4 C-Atome. Alkyl bedeutet vorzugsweise Methyl, weiterhin Ethyl, Propyl, Isopropyl, Butyl, Isobutyl, sek.-Butyl oder tert.-Butyl, ferner auch Pentyl, 1-, 2- oder 3-Methylbutyl, 1,1- , 1,2- oder 2,2-Dimethylpropyl, 1-Ethylpropyl, Hexyl, 1- , 2- , 3- oder 4-Methylpentyl, 1, 1- , 1,2- , 1,3- , 2,2- , 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl.
E bedeutet insbesondere CH₂ oder O, ferner auch S.
D bedeutet insbesondere Carbonyl und CH₂.
X bedeutet vorzugsweise O, ferner bevorzugt S.
m bedeutet insbesondere 0, ferner bevorzugt auch 1 und 2.
n ist vorzugsweise 1, ferner bevorzugt 2 oder 3.
Hal bedeutet vorzugsweise F, Cl oder Br, aber auch I.

Ar ist unsubstituiertes, vorzugsweise - wie angegeben - monosubstituiertes Phenyl, im einzelnen bevorzugt Phenyl, o-, m- oder p-Tolyl, o-, m- oder p-Ethylphenyl, o-, m- oder p-Propylphenyl, o-, m- oder p-Isopropylphenyl, o-, m- oder p-tert.-Butylphenyl, o-, m- oder p-Trifluormethylphenyl, o-, m- oder p-Hydroxyphenyl, o-, m- oder p-Nitrophenyl, o-, m- oder p-Aminophenyl, o-, m- oder p-(N-Methylamino)-phenyl, o-, m- oder p-Acetamidophenyl, o-, m- oder p-(Trifluormethoxy)-phenyl, o-, m- oder p-Cyanphenyl, o-, m- oder p-Methoxyphenyl, o-, m- oder p-Ethoxyphenyl, o-, m- oder p-Carboxyphenyl, o-, m- oder p-Methoxycarbonylphenyl, o-, m- oder p- Ethoxycarbonylphenyl, o-, m- oder p-Benzyloxycarbonylphenyl, o-, m- oder p-(Carboxymethyloxy)-phenyl, o-, m- oder p- (Methoxycarbonyl-methyloxy)-phenyl, o-, m- oder p-(Methoxycarbonyl-ethyloxy)-phenyl, o-, m- oder p-(N,N-Dimethylamino)-phenyl, o-, m- oder p-(N-Ethylamino)-phenyl, o-, m- oder p-(N,N-Diethylamino)-phenyl, o-, m- oder p-Fluorphenyl, o-, m- oder p-Bromphenyl, o-, m- oder p- Chlorphenyl, o-, m- oder p-(Difluormethoxy)-phenyl, o-, m- oder p-(Fluormethoxy)-phenyl, o-, m- oder p-Formylphenyl, o-, m- oder p-Acetylphenyl, o-, m- oder p-Propionylphenyl, o-, m- oder p- Butyrylphenyl, o-, m- oder p-Pentanoylphenyl, o-, m- oder p-( Phenylsulfonamidocarbonyl)-phenyl, o-, m- oder p-Phenoxyphenyl, o-, m- oder p-Methylthiophenyl, o-, m- oder p-Methylsulfinylphenyl, o-, m- oder p-Methylsulfonylphenyl, o-, m- oder p-Benzyloxyphenyl, o-, m- oder p-Cyanmethyloxyphenyl,
weiter bevorzugt 2,3-Methylendioxyphenyl, 3,4-Methylendioxyphenyl, 2,3-Ethylendioxyphenyl, 3,4-Ethylendioxyphenyl, 6-Chlor-3,4-methylendioxyphenyl, 2,3-(2-Oxo-methylendioxy)-phenyl, 2,3-, 2,4- 2,5-, 2,6-, 3,4- oder 3,5-(Difluormethoxy)-(carboxymethyloxy)-phenyl, 2,3-, 2,4- 2,5-, 2,6-, 3,4- oder 3,5-Methoxy-(carboxymethyloxy)-phenyl, 2,3-, 2,4- 2,5-, 2,6-, 3,4- oder 3,5-Hydroxy-(carboxymethyloxy)-phenyl.
Ar bedeutet weiter bevorzugt 2,3-Dihydrobenzofuranyl, 2,3-Dihydro-2-oxo-furanyl, 1,3-Dithiaindanyl, 2,1,3-Benzothiadiazol-4 oder 5-yl, 2,1,3-Benzoxadiazol-4 oder 5-yl,
weiter bevorzugt 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2-Chlor-3-methyl-, 2-Chlor-4-methyl-, 2-Chlor-5-methyl-2-Chlor-6-methyl-, 2-Methyl-3-chlor-, 2-Methyl-4-chlor-, 2-Methyl-5-chlor-, 2-Methyl-6-chlor-, 3-Chlor-4-methyl-, 3-Chlor-5-methyl- oder 3-Methyl-4-chlorphenyl, 2-Brom-3-methyl-, 2-Brom-4-methyl-, 2-Brom-5-methyl-, 2-Brom-6-methyl-, 2-Methyl-3-brom-, 2-Methyl-4-brom-, 2-Methyl-5-brom-, 2-Methyl-6-brom-, 3-Brom-4-methyl-, 3-Brom-5-methyl- oder 3-Methyl-4-bromphenyl, 2,4- oder 2,5-Dinitrophenyl, 2,5- oder 3,4-Dimethoxyphenyl, 3-Nitro-4-chlorphenyl, 2-Amino-3-chlor-, 2-Amino-4-chlor-, 2-Amino-5-chlor- oder 2-Amino-6-chlorphenyl, 2-Nitro-4-N,N-dimethylamino- oder 3-Nitro-4-N,N-dimethylaminophenyl, 3-Carboxy-2-methoxy-, 3-Carboxy-4-methoxy- oder 3-Carboxy-5-methoxyphenyl, 2,3,4-, 2,3,5-, 2,3,6-, 2,4,6- oder 3,4,5-Trichlorphenyl, 2,4,6-tri-tert.-Butylphenyl, ferner bevorzugt 2-Nitro-4-(trifluormethyl)phenyl, 3,5-Di-(trifluormethyl)-phenyl, 2,5-Dimethylphenyl, 2-Hydroxy-3,5-dichlorphenyl, 2-Fluor-5- oder 4-Fluor-3-(trifluormethyl)-phenyl, 4-Chlor-2- oder 4-Chlor-3-(trifluormethyl)-, 2-Chlor-4- oder 2-Chlor-5-(trifluormethyl)-phenyl, 4-Brom-2- oder 4-Brom-3-(trifluormethyl)-phenyl, p-Iodphenyl, 2-Nitro-4-methoxyphenyl, 2,5-Dimethoxy-4-nitrophenyl, 3,5-Dicarboxyphenyl, 2-Chlor-3-nitro-5-carboxy-phenyl, 4-Chlor-3-carboxyphenyl, 2-Methyl-5-nitrophenyl, 2,4-Dimethyl-3-nitrophenyl, 3,6-Dichlor-4-Aminophenyl, 4-Fluor-3-chlorphenyl, 4-Fluor-3,5-dimethylphenyl, 2-Fluor-4-Bromphenyl, 2,5-Difluor-4-bromphenyl, 2,4-Dichlor-5-methylphenyl, 3-Brom-6-methoxyphenyl, 3-Chlor-6-methoxyphenyl, 3-Chlor-4-acetamidophenyl, 4-Hydroxy-3-carboxyphenyl, 2-Methoxy-5-methylphenyl oder 2,4,6-Triisopropylphenyl, ferner Naphthyl.

Der Rest R² bedeutet vorzugsweise Carbomethoxy, Carboethoxy, Carbopropoxy, Carbobutoxy, Carbobenzyloxy, ferner Cyan, 1H-Tetrazol-5-yl oder Phenylsulfonamidocarbonyl, besonders bevorzugt ist aber Carboxy und Carboxymethyl.

Die Reste R³, R⁴ und R⁵ bedeuten jeweils unabhängig voneinander vorzugsweise H, Methyl, Ethyl, Propyl, Isopropyl, Butyl, Isobutyl, sek.-Butyl oder tert.-Butyl, Benzyl, F, Cl, Br, Methoxy, Ethoxy, Phenoxy, Benzyloxy, Nitro oder Cyan, ferner sind bevorzugt Amino, Methylamino, Dimethylamino, Ethylamino, Diethylamino, Acetylamino, Propionylamino, Butyrylamino, Methylsulfonamido, Ethylsulfonamido, Phenylsulfonamido, Methylcarbonyloxy, Ethylcarbonyloxy, Phenylcarbonyloxy, Methyloxycarbonyl, Ethyloxycarbonyl, Methylthio, Ethylthio, Propylthio, Methylsulfinyl, Ethylsulfinyl, Phenylsulfinyl, Methylsulfonyl, Ethylsulfonyl oder Phenylsulfonyl.

Die Verbindungen der Formel I können ein oder mehrere chirale Zentren besitzen und daher in verschiedenen stereoisomeren Formen vorkommen. Die Formel I umschließt alle diese Formen.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln Ia bis Ig ausgedrückt werden, die der Formel I entsprechen und worin die nicht näher bezeichneten Reste die bei der Formel I angegebene Bedeutung haben, worin jedoch
in Ia
   - -Y-Z-: -NR⁷-CO- oder -N=C(OR⁷)- bedeutet;
in Ib
   - -Y-Z-: -N=CR⁸- bedeutet;
in Ic
   - -Y-Z-: -NR⁷-CO- und
   - R²: COOH bedeuten;
in Id
   - -Y-Z-: -NR⁷-CO- und
   - Ar: 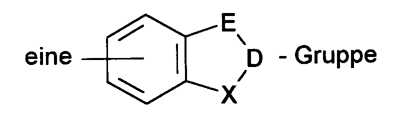 bedeuten;
in Ie
   - -Y-Z-: -N=CR⁸,
   - R⁸: Ar und
   - Ar: ein-, zwei- oder dreifach durch R⁹, R¹⁰ oder R¹¹ substituiertes Phenyl bedeuten;
in If
   - -Y-Z-: -NR⁷-CO-
   - R⁷: CH₂Ar und
   - Ar: 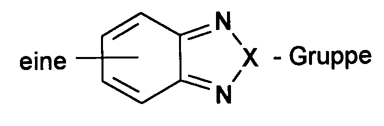 bedeuten;
in Ig
   - -Y-Z-: -N=CR⁸-
   - Ar: 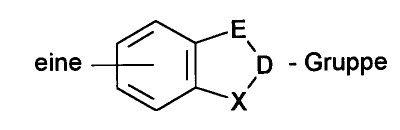 und
   - R⁸: OAr bedeutet.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart; insbesondere aber in der WO 94/14434) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

Verbindungen der Formel I können vorzugsweise erhalten werden, indem man Verbindungen der Formel II mit Verbindungen der Formel III umsetzt.

In den Verbindungen der Formel III bedeutet Q vorzugsweise Cl, Br, I oder eine reaktionsfähig abgewandelte OH-Gruppe wie Alkylsulfonyloxy mit 1-6 C-Atomen (bevorzugt Methylsulfonyloxy) oder Arylsulfonyloxy mit 6-10 C-Atomen (bevorzugt Phenyl- oder p-Tolylsulfonyloxy).

Die Umsetzung erfolgt in der Regel in einem inerten Lösungsmittel, in Gegenwart eines säurebindenden Mittels vorzugsweise eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums, Calciums oder Cäsiums. Auch der Zusatz einer organischen Base wie Triethylamin, Dimethylanilin, Pyridin oder Chinolin oder eines Überschusses der Amidkomponente der Formel II bzw. des Alkylierungsderivates der Formel III kann günstig sein. Die Reaktionszeit liegt je nach den angewendeten Bedingungen zwischen einigen Minuten und 14 Tagen, die Reaktionstemperatur zwischen etwa 0° und 150°, normalerweise zwischen 20° und 130°.

Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.

Die Ausgangsverbindungen der Formel II sind in der Regel neu. Sie können aber nach an sich bekannten Methoden hergestellt werden. So kann z.B. 4-(1,3-Benzodioxol-5-yl)-1,2-dihydro-2-oxochinolin-3-carbonsäuremethylester durch Cyclisierung des bei der Reaktion aus 1-Amino-2-(3,4-methylendioxxbenzoyl)-4-ethoxybenzol und Chlorformylessigsäuremethylester in Gegenwart eines inerten Lösungsmittels und einer Base entstehenden Zwischenproduktes hergestellt werden. Dies erfolgt zweckmäßig bei Temperaturen zwischen 0 und etwa 200°; vorzugsweise arbeitet man zwischen 30 und 80°.
Als inerte Lösungsmittel und als Basen eignen sich die schon oben erwähnten.

Verbindungen der Formel I können weiter bevorzugt erhalten werden, indem man Verbindungen der Formel IV mit Verbindungen der Formel V umsetzt.
In den Verbindungen der Formel IV bedeutet Q vorzugsweise Cl oder Br aber auch I.
In den Verbindungen der Formel V bedeutet L und L' vorzugsweise jeweils unabhängig voneinander OH, Methoxy, Ethoxy, Propoxy oder iso-Propoxy.

Die Umsetzung erfolgt in der Regel in einem inerten Lösungsmittel, in Gegenwart einer Base und eines Edelmetallkatalysators.
Die Reaktionszeit liegt je nach den angewendeten Bedingungen zwischen einigen Minuten und 14 Tagen, die Reaktionstemperatur zwischen etwa 0° und 150°, normalerweise zwischen 20° und 130°.
Als inerte Lösungsmittel und Basen eignen sich die schon oben erwähnten.
Als Edelmetallkatalysatoren sind besonders Palladium-(0)-Katalysatoren bevorzugt, wie Tetrakis(triphenylphosphin)palladium-(0).

Die Ausgangsverbindungen der Formel IV sind in der Regel neu. Sie können aber nach an sich bekannten Methoden hergestellt werden.
So kann z.B. 2-Chlor-4-(4-methoxyphenyl)-chinolin-3-carbonsäuremethylester aus 1,2-Dihydro-4-(4-methoxyphenyl)-2-oxo-chinolin-3-carbonsäuremethylester mit POCl₃ hergestellt werden. Dies erfolgt zweckmäßig bei Temperaturen zwischen 0 und etwa 200°; vorzugsweise arbeitet man zwischen 30 und 80°.

Verbindungen der Formel I können weiter bevorzugt erhalten werden, indem man Verbindungen der Formel IV mit Verbindungen der Formel VI umsetzt.
In den Verbindungen der Formel IV bedeutet Q vorzugsweise Cl, Br, I oder eine reaktionsfähig abgewandelte OH-Gruppe.
Die Umsetzung erfolgt in der Regel in einem inerten Lösungsmittel und unter Zusatz einer der oben erwähnten Basen.

Es ist ferner möglich, eine Verbindung der Formel I in eine andere Verbindung der Formel I umzuwandeln, indem man einen oder mehrere Rest(e) R¹, R², R³, R⁴ und/oder R⁵ in einen oder mehrere andere Reste R¹, R², R³, R⁴ und/oder R⁵ umwandelt, z.B. indem man Nitrogruppen, beispielsweise durch Hydrierung an Raney-Nickel oder Pd-Kohle in einem inerten Lösungsmittel wie Methanol oder Ethanol, zu Aminogruppen reduziert und/oder eine Estergruppe in eine Carboxygruppe umwandelt und/oder eine Aminogruppe durch reduktive Aminierung in ein alkyliertes Amin umwandelt und/oder Carboxygruppen durch Umsetzung mit Alkoholen verestert.

Ferner kann man freie Aminogruppen in üblicher Weise mit einem Säurechlorid oder -anhydrid acylieren oder mit einem unsubstituierten oder substituierten Alkylhalogenid alkylieren, zweckmäßig in einem inerten Lösungsmittel wie Dichlormethan oder THF und /oder in Gegenwart einer Base wie Triethylamin oder Pyridin bei Temperaturen zwischen -60 und +30°.

Gewünschtenfalls kann in einer Verbindung der Formel I eine funktionell abgewandelte Amino- und /oder Hydroxygruppe durch Solvolyse oder Hydrogenolyse nach üblichen Methoden in Freiheit gesetzt werden. So kann z.B. eine Verbindung der Formel I, die eine NHCOR⁶- oder eine COOR⁶-Gruppe enthält, in die entsprechende Verbindung der Formel I umgewandelt werden, die stattdessen eine NH₂- oder eine HOOC-Gruppe enthält. COOR⁶-Gruppen können z.B. mit NaOH oder KOH in Wasser, Wasser-THF oder Wasser-Dioxan bei Temperaturen zwischen 0 und 100° verseift werden.

Eine Base der Formel I kann mit einer Säure in das zugehörige Säureadditionssalz übergeführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und Disulfonsäuren, Laurylschwefelsäure. Salze mit physiologisch nicht unbedenklichen Säuren, z.B. Pikrate, können zur Isolierung und /oder Aufreinigung der Verbindungen der Formel I verwendet werden.

Andererseits können Verbindungen der Formel I mit Basen (z.B. Natrium- oder Kaliumhydroxid oder -carbonat) in die entsprechenden Metall-, insbesondere Alkalimetall- oder Erdalkalimetall-, oder in die entsprechenden Ammoniumsalze umgewandelt werden.

Gegenstand der Erfindung ist ferner die Verwendung der Verbindungen der Formel I und/oder ihrer physiologisch unbedenklichen Salze zur Herstellung pharmazeutischer Zubereitungen, insbesondere auf nichtchemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.

Gegenstand der Erfindung sind ferner pharmazeutische Zubereitungen, enthaltend mindestens eine Verbindung der Formel I und/oder eines ihrer physiologisch unbedenklichen Salze.

Diese Zubereitungen können als Arzneimittel in der Human- oder Veterinärmedizin verwendet werden. Als Trägerstoffe kommen organische oder anorganische Substanzen in Frage, die sich für die enterale (z.B. orale), parenterale oder topische Applikation eignen und mit den neuen Verbindungen nicht reagieren, beispielsweise Wasser, pflanzliche Öle, Benzylalkohole, Alkylenglykole, Polyethylenglykole, Glycerintriacetat, Gelatine, Kohlehydrate wie Lactose oder Stärke, Magnesiumstearat, Talk, Vaseline. Zur oralen Anwendung dienen insbesondere Tabletten, Pillen, Dragees, Kapseln, Pulver, Granulate, Sirupe, Säfte oder Tropfen, zur rektalen Anwendung Suppositorien, zur parenteralen Anwendung Lösungen, vorzugsweise ölige oder wässrige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und /oder mehrere weitere Wirkstoffe enthalten, z.B. ein oder mehrere Vitamine.

Die Verbindungen der Formel I und ihre physiologisch unbedenklichen Salze können bei der Bekämpfung von Krankheiten, insbesondere von Hypertonie und Herzinsuffizienz verwendet werden.

Dabei werden die erfindungsgemäßen Substanzen in der Regel vorzugsweise in Dosierungen zwischen etwa 1 und 500 mg, insbesondere zwischen 5 und 100 mg pro Dosierungseinheit verabreicht. Die tägliche Dosierung ligt vorzugsweise zwischen etwa 0,02 und 10 mg/kg Körpergewicht. Die spezielle Dosis für jeden Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabreichungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel, wobei auch eine Trennung der nachfolgend beschriebenen Isomeren erfolgt, und /oder durch Kristallisation. Rf-Werte an Kieselgel; Laufmittel: Ethylacetat/Methanol 9:1.
- Massenspektrometrie:: EI (Elektronenstoßionisation): M⁺
FAB (Fast Atom Bombardment): (M+H)⁺

### Beispiel 1

Zu einer Lösung von 0,4 g 4-(1,3-Benzodioxol-5-yl)-1,2-dihydro-6-ethoxy-2-oxo-chinolin-3-carbonsäuremethylester (erhältlich durch Reaktion von 1-Amino-2-(3,4-methylendioxybenzoyl)-4-ethoxybenzol und Chlorformylessigsäuremethylester in Dichlormethan und Ethyldiisopropylamin mit anschließender Cyclisierung des Zwischenprodukts in Natriummethanolatlösung, F. 224-225°; 1-Amino-2-(3,4-methylendioxybenzoyl)-4-ethoxybenzol ist erhältlich durch Umsetzung von 3,4-Methylendioxybenzaldehyd und N-(tert.-Butyloxycarbonyl)-p-ethoxyanilin, F. 113-114°, in THF, tert.-Butyllithium bei -78°, anschließender Oxidation des dabei entstehenden 3',4'-Methylendioxy-2-(N-tert.-butyloxycarbonyl-amino)-5-ethoxydiphenylmethanols und Abspaltung der Aminoschutzgruppe, EI-MS 285) in 5 ml DMF und 0,17g Kaliumcarbonat gibt man eine 50 %ige Lösung von 0,4 g 2-Methoxybenzylchlorid ("A") in Dichlormethan. Das Reaktionsgemisch wird 4 Stunden gerührt und wie üblich aufgearbeitet. Man erhält das N-Alkylierungsprodukt 4-(1,3-Benzodioxol-5-yl)-1,2-dihydro-6-ethoxy-1-(2-methoxybenzyl)-2-oxo-chinolin-3-carbonsäuremethylester, F. 182-183° und das O-Alkylierungsprodukt 4-(1,3-Benzodioxol-5-yl)-6-ethoxy-2-(2-methoxybenzyloxy)-chinolin-3-carbonsäuremethylester, F. 157-158°.

Analog erhält man aus folgenden 4-T-1,2-dihydro-6-ethoxy-2-oxo-chinolin-3-carbonsäuremethylestern, worin T
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
mit "A" die nachstehenden 4-T-1,2-dihydro-6-ethoxy-1-(2-methoxybenzyl)-2-oxo-chinolin-3-carbonsäuremethylester, worin T
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
sowie die nachstehenden 4-T-6-ethoxy-2-(2-methoxybenzyloxy)-chinolin-3-carbonsäuremethylester, worin T
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet.

Analog erhält man durch Umsetzung von 3-Methoxybenzylchlorid mit den folgenden 4-T-1,2-dihydro-6-ethoxy-2-oxo-chinolin-3-carbonsäuremethylestern, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
die nachstehenden 4-T-1,2-dihydro-6-ethoxy-1-(3-methoxybenzyl)-2-oxo-chinolin-3-carbonsäuremethylester, worin T
1,3-Benzodioxol-5-yl, F. 138-139°
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
sowie die nachstehenden 4-T-6-ethoxy-2-(3-methoxybenzyloxy)-chinolin-3-carbonsäuremethylester, worin T
1,3-Benzodioxol-5-yl, F. 139-140°
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet.

Analog erhält man durch Umsetzung von 4-Methoxybenzylchlorid mit den folgenden 4-T-1,2-dihydro-6-ethoxy-2-oxo-chinolin-3-carbonsäuremethylestern, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl 2-Hydroxyethyloxy-4-methoxyphenyl
bedeutet,
die nachstehenden 4-T-1,2-dihydro-6-ethoxy-1-(4-methoxybenzyl)-2-oxo-chinolin-3-carbonsäuremethylester, worin T
1,3-Benzodioxol-5-yl, F. 157-158°
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
sowie die nachstehenden 4-T-6-ethoxy-2-(4-methoxybenzyloxy)-chinolin-3-carbonsäuremethylester, worin T
1,3-Benzodioxol-5-yl, F. 141-142°
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet.

Analog erhält man durch Umsetzung von 3,4-Methylendioxybenzylchlorid mit den folgenden 4-T-1,2-dihydro-6-ethoxy-2-oxo-chinolin-3-carbonsäuremethylestern, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
die nachstehenden 4-T-1,2-dihydro-6-ethoxy-1-(3,4-methylendioxybenzyl)-2-oxo-chinolin-3-carbonsäuremethylester, worin T
1,3-Benzodioxol-5-yl, F. 177-178°
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
sowie die nachstehenden 4-T-6-ethoxy-2-(3,4-methylendioxybenzyloxy)-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet.

Analog erhält man durch Umsetzung von 6-Chlor-3,4-methylendioxybenzylchlorid mit den folgenden 4-T-1,2-dihydro-6-ethoxy-2-oxo-chinolin-3-carbonsäuremethylestern, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
die nachstehenden 4-T-1,2-dihydro-6-ethoxy-1-(6-chlor-3,4-methylendioxybenzyl)-2-oxo-chinolin-3-carbonsäuremethylester, worin T
1,3-Benzodioxol-5-yl, F. 207-208°
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
sowie die nachstehenden 4-T-6-ethoxy-2-(6-chlor-3,4-methylendioxybenzyloxy)-chinolin-3-carbonsäuremethylester, worin T
1,3-Benzodioxol-5-yl, F. 200-201°
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet.

Analog erhält man durch Umsetzung von Benzylchlorid mit den folgenden 4-T-1,2-dihydro-6-ethoxy-2-oxo-chinolin-3-carbonsäuremethylestern, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
die nachstehenden 4-T-1,2-dihydro-6-ethoxy-1-benzyl-2-oxo-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
sowie die nachstehenden 4-T-6-ethoxy-2-benzyloxy-chinolin-3-carbonsäuremethylester, worin T
1,3-Benzodioxol-5-yl 4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet.

Analog erhält man durch Umsetzung von 2-(Methylthio)benzylchlorid mit den folgenden 4-T-1,2-dihydro-6-ethoxy-2-oxo-chinolin-3-carbonsäuremethylestern, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
die nachstehenden 4-T-1,2-dihydro-6-ethoxy-1-(2-methylthiobenzyl)-2-oxo-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
sowie die nachstehenden 4-T-6-ethoxy-2-(2-methylthiobenzyloxy)-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet.

Analog erhält man durch Umsetzung von 2-(Methylsulfinyl)benzylchlorid mit den folgenden 4-T-1,2-dihydro-6-ethoxy-2-oxo-chinolin-3-carbonsäuremethylestern, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
die nachstehenden 4-T-1,2-dihydro-6-ethoxy-1-(2-methylsulfinylbenzyl)-2-oxo-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
sowie die nachstehenden 4-T-6-ethoxy-2-(2-methylsulfinylbenzyloxy)-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet.

Analog erhält man durch Umsetzung von 2-(Methylsulfonyl)benzylchlorid mit den folgenden 4-T-1,2-dihydro-6-ethoxy-2-oxo-chinolin-3-carbonsäuremethylestern, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
die nachstehenden 4-T-1,2-dihydro-6-ethoxy-1-(2-methylsulfonylbenzyl)-2-oxo-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
sowie die nachstehenden 4-T-6-ethoxy-2-(2-methylsulfonylbenzyloxy)-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet.

Analog erhält man durch Umsetzung von 2,3-Dimethoxy-benzylchlorid mit den folgenden 4-T-1,2-dihydro-6-ethoxy-2-oxo-chinolin-3-carbonsäuremethylestern, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
die nachstehenden 4-T-1,2-dihydro-6-ethoxy-1-(2,3-dimethoxy-benzyl)-2 oxo-chinolin-3-carbonsäuremethylester, worin T
1,3-Benzodioxol-5-yl, F. 180-181°
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
sowie die nachstehenden 4-T-6-ethoxy-2-(2,3-dimethoxy-benzyloxy)-chinolin-3-carbonsäuremethylester, worin T
1,3-Benzodioxol-5-yl, F. 133-134°
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet.

Analog erhält man durch Umsetzung von 2,3-Methylendioxybenzylchlorid mit den folgenden 4-T-1,2-dihydro-6-ethoxy-2-oxo-chinolin-3-carbonsäuremethylestern, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
die nachstehenden 4-T-1,2-dihydro-6-ethoxy-1-(2,3-methylendioxybenzyl)-2-oxo-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
sowie die nachstehenden 4-T-6-ethoxy-2-(2,3-methylendioxybenzyloxy)-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet.

Analog erhält man durch Umsetzung von 2,1,3-Benzothiadiazol-5-methylchlorid mit den folgenden 4-T-1,2-dihydro-6-ethoxy-2-oxo-chinolin-3-carbonsäuremethylestern, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet
die nachstehenden 4-T-1,2-dihydro-6-ethoxy-1-(2,1,3-benzothiadiazol-5-methyl)-2-oxo-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
sowie die nachstehenden 4-T-6-ethoxy-2-(2,1,3-benzothiadiazol-5-methyloxy)-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet.

Analog erhält man aus folgenden 4-T-1,2-dihydro-2-oxo-chinolin-3-carbonsäuremethylestern, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
mit "A" die nachstehenden 4-T-1,2-dihydro-1-(2-methoxybenzyl)-2-oxo-chinolin-3-carbonsäuremethylester, worin T
1,3-Benzodioxol-5-yl, F. 178-179°
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
sowie die nachstehenden 4-T-2-(2-methoxybenzyloxy)-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet.

Analog erhält man durch Umsetzung von 3-Methoxybenzylchlorid mit den folgenden 4-T-1,2-dihydro-2-oxo-chinolin-3-carbonsäuremethylestern, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
die nachstehenden 4-T-1,2-dihydro-1-(3-methoxybenzyl)-2-oxo-chinolin-3-carbonsäuremethylester, worin T
1,3-Benzodioxol-5-yl, F. 162-163°
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
sowie die nachstehenden 4-T-2-(3-methoxybenzyloxy)-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet.

Analog erhält man durch Umsetzung von 4-Methoxybenzylchlorid mit den folgenden 4-T-1,2-dihydro-2-oxo-chinolin-3-carbonsäuremethylestern, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
die nachstehenden 4-T-1,2-dihydro-1-(4-methoxybenzyl)-2-oxo-chinolin-3-carbonsäuremethylester, worin T
1,3-Benzodioxol-5-yl, F. 214-215°
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
sowie die nachstehenden 4-T-2-(4-methoxybenzyloxy)-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet.

Analog erhält man durch Umsetzung von 3,4-Methylendioxybenzylchlorid mit den folgenden 4-T-1,2-dihydro-2-oxo-chinolin-3-carbonsäuremethylestern, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
die nachstehenden 4-T-1,2-dihydro-1-(3,4-methylendioxybenzyl)-2-oxo-chinolin-3-carbonsäuremethylester, worin T
1,3-Benzodioxol-5-yl, F. 192-193°
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
sowie die nachstehenden 4-T-2-(3,4-methylendioxybenzyloxy)-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet.

Analog erhält man durch Umsetzung von 6-Chlor-3,4-methylendioxybenzylchlorid mit den folgenden 4-T-1,2-dihydro-2-oxo-chinolin-3-carbonsäuremethylestern, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
die nachstehenden 4-T-1,2-dihydro-1-(6-chlor-3,4-methylendioxybenzyl)-2-oxo-chinolin-3-carbonsäuremethylester, worin T
1,3-Benzodioxol-5yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
sowie die nachstehenden 4-T-2-(6-chlor-3,4-methylendioxybenzyloxy)-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet.

Analog erhält man durch Umsetzung von Benzylchlorid mit den folgenden 4-T-1,2-dihydro-2-oxo-chinolin-3-carbonsäuremethylestern, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
die nachstehenden 4-T-1,2-dihydro-1-benzyl-2-oxo-chinolin-3-carbonsäuremethylester, worin T
1,3-Benzodioxol-5-yl, F. 139-140°
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
sowie die nachstehenden 4-T-2-benzyloxy-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet.

Analog erhält man durch Umsetzung von 2-Methylthiobenzylchlorid mit den folgenden 4-T-1,2-dihydro-2-oxo-chinolin-3-carbonsäuremethylestern, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
die nachstehenden 4-T-1,2-dihydro-1-(2-methylthiobenzyl)-2-oxo-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
sowie die nachstehenden 4-T-2-(2-methylthiobenzyloxy)-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet.

Analog erhält man durch Umsetzung von 2-Methylsulfinylbenzylchlorid mit den folgenden 4-T-1,2-dihydro-2-oxo-chinolin-3-carbonsäuremethylestern, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
die nachstehenden 4-T-1,2-dihydro-1-(2-methylsulfinylbenzyl)-2-oxo-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
sowie die nachstehenden 4-T-2-(2-methylsulfinylbenzyloxy)-chinolin-3-carbonsäuremethylester, worin T
1,3-Benzodioxol-5-yl
4-Methoxyphenyl
2-4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet.

Analog erhält man durch Umsetzung von 2-Methylsulfonylbenzylchlorid mit den folgenden 4-T-1,2-dihydro-2-oxo-chinolin-3-carbonsäuremethylestern, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
die nachstehenden 4-T-1,2-dihydro-1-(2-methylsulfonylbenzyl)-2-oxo-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
sowie die nachstehenden 4-T-2-(2-methylsulfonylbenzyloxy)-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet.

Analog erhält man durch Umsetzung von 2,3-Dimethoxy-benzylchlorid mit den folgenden 4-T-1,2-dihydro-2-oxo-chinolin-3-carbonsäuremethylestern, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
die nachstehenden 4-T-1,2-dihydro-1-(2,3-dimethoxy-benzyl)-2-oxo-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
sowie die nachstehenden 4-T-2-(2,3-dimethoxy-benzyloxy)-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet.

Analog erhält man durch Umsetzung von 2,3-Methylendioxybenzylchlorid mit den folgenden 4-T-1,2-dihydro-2-oxo-chinolin-3-carbonsäuremethylestern, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
die nachstehenden 4-T-1,2-dihydro-1-(2,3-methylendioxybenzyl)-2-oxo-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
sowie die nachstehenden 4-T-2-(2,3-methylendioxy-benzyloxy)-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet.

Analog erhält man aus folgenden 4-T-1,2-dihydro-6-propionyl-2-oxo-chinolin-3-carbonsäuremethylestern, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
mit "A" die nachstehenden 4-T-1,2-dihydro-1-(2-methoxybenzyl)-6-propionyl-2-oxo-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
sowie die nachstehenden 4-T-2-(2-methoxybenzyloxy)-6-propionyl-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet.

Analog erhält man durch Umsetzung von 3-Methoxybenzylchlorid mit den folgenden 4-T-1,2-dihydro-6-propionyl-2-oxo-chinolin-3-carbonsäuremethylestern, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
die nachstehenden 4-T-1,2-dihydro-1-(3-methoxybenzyl)-6-propionyl-2-oxo-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
sowie die nachstehenden 4-T-2-(3-methoxybenzyloxy)-6-propionyl-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet.

Analog erhält man durch Umsetzung von 4-Methoxybenzylchlorid mit den folgenden 4-T-1,2-dihydro-6-propionyl-2-oxo-chinolin-3-carbonsäuremethylestern, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
die nachstehenden 4-T-1,2-dihydro-1-(4-methoxybenzyl)-6-propionyl-2-oxo-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
sowie die nachstehenden 4-T-2-(4-methoxybenzyloxy)-6-propionyl-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet.

Analog erhält man durch Umsetzung von 3,4-Methylendioxybenzylchlorid mit den folgenden 4-T-1,2-dihydro-6-propionyl-2-oxo-chinolin-3-carbonsäuremethylestern, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
die nachstehenden 4-T-1,2-dihydro-1-(3,4-methylendioxybenzyl)-6-propionyl-2-oxo-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
sowie die nachstehenden 4-T-2-(3,4-methylendioxybenzyloxy)-6-propionyl-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet.

Analog erhält man durch Umsetzung von 6-Chlor-3,4-methylendioxybenzylchlorid mit den folgenden 4-T-1,2-dihydro-6-propionyl-2-oxo-chinolin-3-carbonsäuremethylestern, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
die nachstehenden 4-T-1,2-dihydro-1-(6-chlor-3,4-methylendioxybenzyl)-6-propionyl-2-oxo-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
sowie die nachstehenden 4-T-2-(6-chlor-3,4-methylendioxybenzyloxy)-6-propionyl-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet.

Analog erhält man durch Umsetzung von Benzylchlorid mit den folgenden 4-T-1,2-dihydro-6-propionyl-2-oxo-chinolin-3-carbonsäuremethylestern, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
die nachstehenden 4-T-1,2-dihydro-1-benzyl-6-propionyl-2-oxo-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
sowie die nachstehenden 4-T-2-benzyloxy-6-propionyl-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet.

Analog erhält man aus folgenden 4-T-1,2-dihydro-6,7-dimethoxy-2-oxo-chinolin-3-carbonsäuremethylestern, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
mit "A" die nachstehenden 4-T-1,2-dihydro-6,7-dimethoxy-1-(2-methoxybenzyl)-2-oxo-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
sowie die nachstehenden 4-T-6,7-dimethoxy-2-(2-methoxybenzyloxy)-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet.

Analog erhält man durch Umsetzung von 3-Methoxybenzylchlorid mit den folgenden 4-T-1,2-dihydro-6,7-dimethoxy-2-oxo-chinolin-3-carbonsäuremethylestern, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
die nachstehenden 4-T-1,2-dihydro-6,7-dimethoxy-1-(3-methoxybenzyl)-2-oxo-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
sowie die nachstehenden 4-T-6,7-dimethoxy-2-(3-methoxybenzyloxy)-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet.

Analog erhält man durch Umsetzung von 4-Methoxybenzylchlorid mit den folgenden 4-T-1,2-dihydro-6,7-dimethoxy-2-oxo-chinolin-3-carbonsäuremethylestern, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
die nachstehenden 4-T-1,2-dihydro-6,7-dimethoxy-1-(4-methoxybenzyl)-2-oxo-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
sowie die nachstehenden 4-T-6,7-dimethoxy-2-(4-methoxybenzyloxy)-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet.

Analog erhält man durch Umsetzung von 3,4-Methylendioxybenzylchlorid mit den folgenden 4-T-1,2-dihydro-6,7-dimethoxy-2-oxo-chinolin-3-carbonsäuremethylestern, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
die nachstehenden 4-T-1,2-dihydro-6,7-dimethoxy-1-(3,4-methylendioxybenzyl)-2-oxo-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
sowie die nachstehenden 4-T-6,7-dimethoxy-2-(3,4-methylendioxybenzyloxy)-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet.

Analog erhält man durch Umsetzung von 6-Chlor-3,4-methylendioxybenzylchlorid mit den folgenden 4-T-1,2-dihydro-6,7-dimethoxy-2-oxo-chinolin-3-carbonsäuremethylestern, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
die nachstehenden 4-T-1,2-dihydro-6,7-dimethoxy-1-(6-chlor-3,4-methylendioxybenzyl)-2-oxo-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
sowie die nachstehenden 4-T-6,7-dimethoxy-2-(6-chlor-3,4-methylendioxybenzyloxy)-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet.

Analog erhält man durch Umsetzung von Benzylchlorid mit den folgenden 4-T-1,2-dihydro-6,7-dimethoxy-2-oxo-chinolin-3-carbonsäuremethylestern, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
die nachstehenden 4-T-1,2-dihydro-6,7-dimethoxy-1-benzyl-2-oxo-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
sowie die nachstehenden 4-T-2-benzyloxy-6,7-dimethoxy-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet.

Analog erhält man aus folgenden 4-T-1,2-dihydro-6,7-dimethyl-2-oxo-chinolin-3-carbonsäuremethylestern, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
mit "A" die nachstehenden 4-T-1,2-dihydro-6,7-dimethyl-1-(2-methoxybenzyl)-2-oxo-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
sowie die nachstehenden 4-T-6,7-dimethyl-2-(2-methoxybenzyloxy)-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet.

Analog erhält man durch Umsetzung von 3-Methoxybenzylchlorid mit den folgenden 4-T-1,2-dihydro-6,7-dimethyl-2-oxo-chinolin-3-carbonsäuremethylestern, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
die nachstehenden 4-T-1,2-dihydro-6,7-dimethyl-1-(3-methoxybenzyl)-2-oxo-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
sowie die nachstehenden 4-T-6,7-dimethyl-2-(3-methoxybenzyloxy)-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet.

Analog erhält man durch Umsetzung von 4-Methoxybenzylchlorid mit den folgenden 4-T-1,2-dihydro-6,7-dimethyl-2-oxo-chinolin-3-carbonsäuremethylestern, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
die nachstehenden 4-T-1,2-dihydro-6,7-dimethyl-1-(4-methoxybenzyl)-2-oxo-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
sowie die nachstehenden 4-T-6,7-dimethyl-2-(4-methoxybenzyloxy)-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet.

Analog erhält man durch Umsetzung von 3,4-Methylendioxybenzylchlorid mit den folgenden 4-T-1,2-dihydro-6,7-dimethyl-2-oxo-chinolin-3-carbonsäuremethylestern, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
die nachstehenden 4-T-1,2-dihydro-6,7-dimethyl-1-(3,4-methylendioxybenzyl)-2-oxo-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
sowie die nachstehenden 4-T-6,7-dimethyl-2-(3,4-methylendioxybenzyloxy)-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet.

Analog erhält man durch Umsetzung von 6-Chlor-3,4-methylendioxybenzylchlorid mit den folgenden 4-T-1,2-dihydro-6,7-dimethyl-2-oxo-chinolin-3-carbonsäuremethylestern, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
die nachstehenden 4-T-1,2-dihydro-6,7-dimethyl-1-(6-chlor-3,4-methylendioxybenzyl)-2-oxo-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
sowie die nachstehenden 4-T-6,7-dimethyl-2-(6-chlor-3,4-methylendioxybenzyloxy)-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet.

Analog erhält man durch Umsetzung von Benzylchlorid mit den folgenden 4-T-1,2-dihydro-6,7-dimethyl-2-oxo-chinolin-3-carbonsäuremethylestern, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
die nachstehenden 4-T-1,2-dihydro-6,7-dimethyl-1-benzyl-2-oxo-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
sowie die nachstehenden 4-T-2-benzyloxy-6,7-dimethyl-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet.

Analog erhält man aus folgenden 4-T-1,2-dihydro-6-ethyl-2-oxo-chinolin-3-carbonsäuremethylestern, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
mit "A" die nachstehenden 4-T-1,2-dihydro-6-ethyl-1-(2-methoxybenzyl)-2-oxo-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
sowie die nachstehenden 4-T-6-ethyl-2-(2-methoxybenzyloxy)-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet.

Analog erhält man durch Umsetzung von 3-Methoxybenzylchlorid mit den folgenden 4-T-1,2-dihydro-6-ethyl-2-oxo-chinolin-3-carbonsäuremethylestern, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
die nachstehenden 4-T-1,2-dihydro-6-ethyl-1-(3-methoxybenzyl)-2-oxo-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
sowie die nachstehenden 4-T-6-ethyl-2-(3-methoxybenzyloxy)-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet.

Analog erhält man durch Umsetzung von 4-Methoxybenzylchlorid mit den folgenden 4-T-1,2-dihydro-6-ethyl-2-oxo-chinolin-3-carbonsäuremethylestern, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
die nachstehenden 4-T-1,2-dihydro-6-ethyl-1-(4-methoxybenzyl)-2-oxo-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
sowie die nachstehenden 4-T-6-ethyl-2-(4-methoxybenzyloxy)-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet.

Analog erhält man durch Umsetzung von 3,4-Methylendioxybenzylchlorid mit den folgenden 4-T-1,2-dihydro-6-ethyl-2-oxo-chinolin-3-carbonsäuremethylestern, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
die nachstehenden 4-T-1,2-dihydro-6-ethyl-1-(3,4-methylendioxybenzyl)-2-oxo-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
sowie die nachstehenden 4-T-6-ethyl-2-(3,4-methylendioxybenzyloxy)-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet.

Analog erhält man durch Umsetzung von 6-Chlor-3,4-methylendioxybenzylchlorid mit den folgenden 4-T-1,2-dihydro-6-ethyl-2-oxo-chinolin-3-carbonsäuremethylestern, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
die nachstehenden 4-T-1,2-dihydro-6-ethyl-1-(6-chlor-3,4-methylendioxybenzyl)-2-oxo-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
sowie die nachstehenden 4-T-6-ethyl-2-(6-chlor-3,4-methylendioxybenzyloxy)-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet.

Analog erhält man durch Umsetzung von Benzylchlorid mit den folgenden 4-T-1,2-dihydro-6-ethyl-2-oxo-chinolin-3-carbonsäuremethylestern, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
die nachstehenden 4-T-1,2-dihydro-6-ethyl-1-benzyl-2-oxo-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
sowie die nachstehenden 4-T-6-ethyl-2-benzyloxy-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet.

Analog erhält man durch Umsetzung von 2-Methylthiobenzylchlorid mit den folgenden 4-T-1,2-dihydro-6-ethyl-2-oxo-chinolin-3-carbonsäuremethylestern, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
die nachstehenden 4-T-1,2-dihydro-6-ethyl-1-(2-methylthiobenzyl)-2-oxo-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
sowie die nachstehenden 4-T-6-ethyl-2-(2-methylthiobenzyloxy)-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet.

Analog erhält man durch Umsetzung von 2-Methylsulfinylbenzylchlorid mit den folgenden 4-T-1,2-dihydro-6-ethyl-2-oxo-chinolin-3-carbonsäuremethylestern, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
die nachstehenden 4-T-1,2-dihydro-6-ethyl-1-(2-methylsulfinylbenzyl)-2-oxo-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
sowie die nachstehenden 4-T-6-ethyl-2-(2-methylsulfinylbenzyloxy)-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet.

Analog erhält man durch Umsetzung von 2-Methylsulfonylbenzylchlorid mit den folgenden 4-T-1,2-dihydro-6-ethyl-2-oxo-chinolin-3-carbonsäuremethylestern, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
die nachstehenden 4-T-1,2-dihydro-6-ethyl-1-(2-methylsulfonylbenzyl)-2-oxo-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
sowie die nachstehenden 4-T-6-ethyl-2-(2-methylsulfonylbenzyloxy)-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet.

Analog erhält man durch Umsetzung von 2,3-Dimethoxy-benzylchlorid mit den folgenden 4-T-6-ethyl-1,2-dihydro-2-oxo-chinolin-3-carbonsäuremethylestern, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
die nachstehenden 4-T-1,2-dihydro-6-ethyl-1-(2,3-dimethoxy-benzyl)-2-oxo-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
sowie die nachstehenden 4-T-6-ethyl-2-(2,3-dimethoxy-benzyloxy)-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet.

Analog erhält man durch Umsetzung von 2,3-Methylendioxybenzylchlorid mit den folgenden 4-T-6-ethyl-1,2-dihydro-2-oxo-chinolin-3-carbonsäuremethylestern, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
die nachstehenden 4-T-1,2-dihydro-6-ethyl-1-(2,3-methylendioxybenzyl)-2-oxo-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
sowie die nachstehenden 4-T-6-ethyl-2-(2,3-methylendioxy-benzyloxy)-chinolin-3-carbonsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet.

### Beispiel 2

Eine Lösung von 0,5 g 4-(1,3-Benzodioxol-5-yl)-1,2-dihydro-6-ethoxy-1-(2-methoxybenzyl)-2-oxo-chinolin-3-carbonsäuremethylester und 6 ml 5N KOH in 10 ml Methanol werden 2 Stunden unter Rückfluß gekocht. Man arbeitet wie üblich auf und erhält 4-(1,3-Benzodioxol-5-yl)-1,2-dihydro-6-ethoxy-1-(2-methoxybenzyl)-2-oxo-chinolin-3-carbonsäure, F. 225-226°. Analog erhält man aus 4-(1,3-Benzodioxol-5-yl)-6-ethoxy-2-(2-methoxybenzyloxy)-chinolin-3-carbonsäuremethylester das O-Alkylierungsprodukt 4-(1,3-Benzodioxol-5-yl)-6-ethoxy-2-(2-methoxybenzyloxy)-chinolin-3-carbonsäure.

Analog erhält man aus den nachstehenden
4-T-1,2-dihydro-6-ethoxy-1-(2-methoxybenzyl)-2-oxo-chinolin-3-carbonsäuremethylestern und
4-T-6-ethoxy-2-(2-methoxybenzyloxy)-chinolin-3-carbonsäuremethylestern,
   worin T
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet
und den
4-T-1,2-dihydro-6-ethoxy-1-(3-methoxybenzyl)-2-oxo-chinolin-3-carbonsäuremethylestern,
4-T-6-ethoxy-2-(3-methoxybenzyloxy)-chinolin-3-carbonsäuremethylestern,
4-T-1,2-dihydro-6-ethoxy-1-(4-methoxybenzyl)-2-oxo-chinolin-3-carbonsäuremethylestern,
4-T-6-ethoxy-2-(4-methoxybenzyloxy)-chinolin-3-carbonsäuremethylestern,
4-T-1,2-dihydro-6-ethoxy-1-(3,4-methylendioxybenzyl)-2-oxo-chinolin-3-carbonsäuremethylestern,
4-T-6-ethoxy-2-(3,4-methylendioxybenzyloxy)-chinolin-3-carbonsäuremethylestern,
4-T-1,2-dihydro-6-ethoxy-1-(6-chlor-3,4-methylendioxybenzyl)-2-oxo-chinolin-3-carbonsäuremethylestern,
4-T-6-ethoxy-2-(6-chlor-3,4-methylendioxybenzyloxy)-chinolin-3-carbonsäuremethylestern,
4-T-1,2-dihydro-6-ethoxy-1-benzyl-2-oxo-chinolin-3-carbonsäuremethylestern,
4-T-6-ethoxy-2-benzyloxy-chinolin-3-carbonsäuremethylestern,
4-T-1,2-dihydro-6-ethoxy-1-(2-methylthiobenzyl)-2-oxo-chinolin-3-carbonsäuremethylestern,
4-T-6-ethoxy-2-(2-methylthiobenzyloxy)-chinolin-3-carbonsäuremethylestern,
4-T-1,2-dihydro-6-ethoxy-1-(2-methylsulfinylbenzyl)-2-oxo-chinolin-3-carbonsäuremethylestern,
4-T-6-ethoxy-2-(2-methylsulfinylbenzyloxy)-chinolin-3-carbonsäuremethylestern,
4-T-1,2-dihydro-6-ethoxy-1-(2-methylsulfonylbenzyl)-2-oxo-chinolin-3-carbonsäuremethylestern,
4-T-6-ethoxy-2-(2-methylsulfonylbenzyloxy)-chinolin-3-carbonsäuremethylestern,
4-T-1,2-dihydro-6-ethoxy-1-(2,3-dimethoxy-benzyl)-2-oxo-chinolin-3-carbonsäuremethylestern,
4-T-6-ethoxy-2-(2,3-dimethoxy-benzyloxy)-chinolin-3-carbonsäuremethylestern,
4-T-1,2-dihydro-6-ethoxy-1-(2,3-methylendioxybenzyl)-2-oxo-chinolin-3-carbonsäuremethylestern,
4-T-6-ethoxy-2-(2,3-methylendioxybenzyloxy)-chinolin-3-carbonsäuremethylestern,
4-T-1,2-dihydro-6-ethoxy-1-(2,1,3-benzothiadiazol-5-methyl)-2-oxo-chinolin-3-carbonsäuremethylestern,
4-T-6-ethoxy-2-(2,1,3-benzothiadiazol-5-methyloxy)-chinolin-3-carbonsäuremethylestern,
4-T-1,2-dihydro-1-(2-methoxybenzyl)-2-oxo-chinolin-3-carbonsäuremethylestern,
4-T-2-(2-methoxybenzyloxy)-chinolin-3-carbonsäuremethylestern,
4-T-1,2-dihydro-1-(3-methoxybenzyl)-2-oxo-chinolin-3-carbonsäuremethylestern,
4-T-2-(3-methoxybenzyloxy)-chinolin-3-carbonsäuremethylestern,
4-T-1,2-dihydro-1-(4-methoxybenzyl)-2-oxo-chinolin-3-carbonsäuremethylestern,
4-T-2-(4-methoxybenzyloxy)-chinolin-3-carbonsäuremethylestern,
4-T-1,2-dihydro-1-(3,4-methylendioxybenzyl)-2-oxo-chinolin-3-carbonsäuremethylestern,
4-T-2-(3,4-methylendioxybenzyloxy)-chinolin-3-carbonsäuremethylestern,
4-T-1,2-dihydro-1-(6-chlor-3,4-methylendioxybenzyl)-2-oxo-chinolin-3-carbonsäuremethylestern,
4-T-2-(6-chlor-3,4-methylendioxybenzyloxy)-chinolin-3-carbonsäuremethylestern,
4-T-1,2-dihydro-1-benzyl-2-oxo-chinolin-3-carbonsäuremethylestern,
4-T-2-benzyloxy-chinolin-3-carbonsäuremethylestern,
4-T-1,2-dihydro-1-(2-methylthiobenzyl)-2-oxo-chinolin-3-carbonsäuremethylestern,
4-T-2-(2-methylthiobenzyloxy)-chinolin-3-carbonsäuremethylestern,
4-T-1,2-dihydro-1-(2-methylsulfinylbenzyl)-2-oxo-chinolin-3-carbonsäuremethylestern,
4-T-2-(2-methylsulfinylbenzyloxy)-chinolin-3-carbonsäuremethylestern,
4-T-1,2-dihydro-1-(2-methylsulfonylbenzyl)-2-oxo-chinolin-3-carbonsäuremethylestern,
4-T-2-(2-methylsulfonylbenzyloxy)-chinolin-3-carbonsäuremethylestern,
4-T-1,2-dihydro-1-(2,3-dimethoxy-benzyl)-2-oxo-chinolin-3-carbonsäuremethylestern,
4-T-2-(2,3-dimethoxy-benzyloxy)-chinolin-3-carbonsäuremethylestern,
4-T-1,2-dihydro-1-(2,3-methylendioxybenzyl)-2-oxo-chinolin-3-carbonsäuremethylestern,
4-T-2-(2,3-methylendoxybenzyloxy)-chinolin-3-carbonsäuremethylestern,
4-T-1,2-dihydro-1-(2,1,3-benzothiadiazol-5-methyl)-2-oxo-chinolin-3-carbonsäuremethylestern,
4-T-2-(2,1,3-benzothiadiazol-5-methyloxy)-chinolin-3-carbonsäuremethylestern,
4-T-1,2-dihydro-1-(2-methoxybenzyl)-6-propionyl-2-oxo-chinolin-3-carbonsäuremethylestern,
4-T-2-(2-methoxybenzyloxy)-6-propionyl-chinolin-3-carbonsäuremethylestern,
4-T-1,2-dihydro-1-(3-methoxybenzyl)-6-propionyl-2-oxo-chinolin-3-carbonsäuremethylestern,
4-T-2-(3-methoxybenzyloxy)-6-propionyl-chinolin-3-carbonsäuremethylestern,
4-T-1,2-dihydro-1-(4-methoxybenzyl)-6-propionyl-2-oxo-chinolin-3-carbonsäuremethylestern,
4-T-2-(4-methoxybenzyloxy)-6-propionyl-chinolin-3-carbonsäuremethylestern,
4-T-1,2-dihydro-1-(3,4-methylendioxybenzyl)-6-propionyl-2-oxo-chinolin-3-carbonsäuremethylestern,
4-T-2-(3,4-methylendioxybenzyloxy)-6-propionyl-chinolin-3-carbonsäuremethylestern,
4-T-1,2-dihydro-1-(6-chlor-3,4-methylendioxybenzyl)-6-propionyl-2-oxo-chinolin-3-carbonsäuremethylestern,
4-T-2-(6-chor-3,4-methylendioxybenzyloxy)-6-propionyl-chinolin-3-carbonsäuremethylestern,
4-T-1,2-dihydro-1-benzyl-6-propionyl-2-oxo-chinolin-3-carbonsäuremethylestern,
4-T-2-benzyloxy-6-propionyl-chinolin-3-carbonsäuremethylestern,
4-T-1,2-dihydro-6,7-dimethoxy-1-(2-methoxybenzyl)-2-oxo-chinolin-3-carbonsäuremethylestern,
4-T-6,7-dimethoxy-2-(2-methoxybenzyloxy)-chinolin-3-carbonsäuremethylestern,
4-T-1,2-dihydro-6,7-dimethoxy-1-(3-methoxybenzyl)-2-oxo-chinolin-3-carbonsäuremethylestern,
4-T-6,7-dimethoxy-2-(3-methoxybenzyloxy)-chinolin-3-carbonsäuremethylestern,
4-T-1,2-dihydro-6,7-dimethoxy-1-(4-methoxybenzyl)-2-oxo-chinolin-3-carbonsäuremethylestern,
4-T-6,7-dimethoxy-2-(4-methoxybenzyloxy)-chinolin-3-carbonsäuremethylestern,
4-T-1,2-dihydro-6,7-dimethoxy-1-(3,4-methylendioxybenzyl)-2-oxo-chinolin-3-carbonsäuremethylestern,
4-T-6,7-dimethoxy-2-(3,4-methylendioxybenzyloxy)-chinolin-3-carbonsäuremethylestern,
4-T-1,2-dihydro-6,7-dimethoxy-1-(6-chlor-3,4-methylendioxybenzyl)-2-oxo-chinolin-3-carbonsäuremethylestern,
4-T-6,7-dimethoxy-2-(6-chlor-3,4-methylendioxybenzyloxy)-chinolin-3-carbonsäuremethylestern,
4-T-1,2-dihydro-6,7-dimethoxy-1-benzyl-2-oxo-chinolin-3-carbonsäuremethylestern,
4-T-2-benzyloxy-6,7-dimethoxy-chinolin-3-carbonsäuremethylestern,
4-T-1,2-dihydro-6,7-dimethyl-1(2-methoxybenzyl)-2-oxo-chinolin-3-carbonsäuremethylestern,
4-T-6,7-dimethyl-2-(2-methoxybenzyloxy)-chinolin-3-carbonsäuremethylestern,
4-T-1,2-dihydro-6,7-dimethyl-1-(3-methoxybenzyl)-2-oxo-chinolin-3-carbonsäuremethylestern,
4-T-6,7-dimethyl-2-(3-methoxybenzyloxy)-chinolin-3-carbonsäuremethylestern,
4-T-1,2-dihydro-6,7-dimethyl-1-(4-methoxybenzyl)-2-oxo-chinolin-3-carbonsäuremethylestern,
4-T-6,7-dimethyl-2-(4-methoxybenzyloxy)-chinolin-3-carbonsäuremethylestern,
4-T-1,2-dihydro-6,7-dimethyl-1-(3,4-methylendioxybenzyl)-2-oxo-chinolin-3-carbonsäuremethylestern,
4-T-6,7-dimethyl-2-(3,4-methylendioxybenzyloxy)-chinolin-3-carbonsäuremethylestern,
4-T-1,2-dihydro-6,7-dimethyl-1-(6-chlor-3,4-methylendioxybenzyl)-2-oxo-chinolin-3-carbonsäuremethylestern,
4-T-6,7-dimethyl-2-(6-chlor-3,4-methylendioxybenzyloxy)-chinolin-3-carbonsäuremethylestern,
4-T-1,2-dihydro-6,7-dimethyl-1-benzyl-2-oxo-chinolin-3-carbonsäuremethylestern,
4-T-2-benzyloxy-6,7-dimethyl-chinolin-3-carbonsäuremethylestern,
4-T-1,2-dihydro-6-ethyl-1-(2-methoxybenzyl)-2-oxo-chinolin-3-carbonsäuremethylestern,
4-T-6-ethyl-2-(2-methoxybenzyloxy)-chinolin-3-carbonsäuremethylestern,
4-T-1,2-dihydro-6-ethyl-1-(3-methoxybenzyl)-2-oxo-chinolin-3-carbonsäuremethylestern,
4-T-6-ethyl-2-(3-methoxybenzyloxy)-chinolin-3-carbonsäuremethylestern,
4-T-1,2-dihydro-6-ethyl-1-(4-methoxybenzyl)-2-oxo-chinolin-3-carbonsäuremethylestern,
4-T-6-ethyl-2-(4-methoxybenzyloxy)-chinolin-3-carbonsäuremethylestern,
4-T-1,2-dihydro-6-ethyl-1-(3,4-methylendioxybenzyl)-2-oxo-chinolin-3-carbonsäuremethylestern,
4-T-6-ethyl-2-(3,4-methylendioxybenzyloxy)-chinolin-3-carbonsäuremethylestern,
4-T-1,2-dihydro-6-ethyl-1-(6-chlor-3,4-methylendioxybenzyl)-2-oxo-chinolin-3-carbonsäuremethylestern,
4-T-6-ethyl-2-(6-chlor-3,4-methylendioxybenzyloxy)-chinolin-3-carbonsäuremethylestern,
4-T-1,2-dihydro-6-ethyl-1-benzyl-2-oxo-chinolin-3-carbonsäuremethylestern,
4-T-6-ethyl-2-benzyloxy-chinolin-3-carbonsäuremethylestern,
4-T-1,2-dihydro-6-ethyl-1-(2-methylthiobenzyl)-2-oxo-chinolin-3-carbonsäuremethylestern,
4-T-6-ethyl-2-(2-methylthiobenzyloxy)-chinolin-3-carbonsäuremethylestern,
4-T-1,2-dihydro-6-ethyl-1-(2-methylsulfinylbenzyl)-2-oxo-chinolin-3-carbonsäuremethylestern,
4-T-6-ethyl-2-(2-methylsulfinylbenzyloxy)-chinolin-3-carbonsäuremethylestern,
4-T-1,2-dihydro-6-ethyl-1-(2-methylsulfonylbenzyl)-2-oxo-chinolin-3-carbonsäuremethylestern,
4-T-6-ethyl-2-(2-methylsulfonylbenzyloxy)-chinolin-3-carbonsäuremethylestern,
4-T-1,2-dihydro-6-ethyl-1-(2,3-dimethoxy-benzyl)-2-oxo-chinolin-3-carbonsäuremethylestern,
4-T-6-ethyl-2-(2,3-dimethoxy-benzyloxy)-chinolin-3-carbonsäuremethylestern,
4-T-1,2-dihydro-6-ethyl-1-(2,3-methylendioxybenzyl)-2-oxo-chinolin-3-carbonsäuremethylestern,
4-T-6-ethyl-2-(2,3-methylendioxybenzyloxy)-chinolin-3-carbonsäuremethylestern,
4-T-1,2-dihydro-6-ethyl-1-(2,1,3-benzothiadiazol-5-methyl)-2-oxo-chinolin-3-carbonsäuremethylestern und
4-T-6-ethyl-2-(2,1,3-benzothiadiazol-5-methyloxy)-chinolin-3-carbonsäuremethylestern,
   worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
die nachfolgenden
4-T-1,2-dihydro-6-ethoxy-1-(2-methoxybenzyl)-2-oxo-chinolin-3-carbonsäuren, worin T
   4-Methoxyphenyl, F. 234,5°
   2,4-Dimethoxyphenyl, F. 220-221°
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-6-ethoxy-2-(2-methoxybenzyloxy)-chinolin-3-carbonsäuren, worin T
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-1,2-dihydro-6-ethoxy-1-(3-methoxybenzyl)-2-oxo-chinolin-3-carbonsäuren, worin T
   1,3-Benzodioxol-5-yl, F. 174-175°
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-6-ethoxy-2-(3-methoxybenzyloxy)-chinolin-3-carbonsäuren, worin T
   1,3-Benzodioxol-5-yl, F. 53-54°
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-1,2-dihydro-6-ethoxy-1-(4-methoxybenzyl)-2-oxo-chinolin-3-carbonsäuren, worin T
   1,3-Benzodioxol-5-yl, F.209-210°
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-6-ethoxy-2-(4-methoxybenzyloxy)-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-1,2-dihydro-6-ethoxy-1-(3,4-methylendioxybenzyl)-2-oxo-chinolin-3-carbonsäuren, worin T
   1,3-Benzodioxol-5-yl, F. 210-111°
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-6-ethoxy-2-(3,4-methylendioxybenzyloxy)-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-1,2-dihydro-6-ethoxy-1-(6-chlor-3,4-methylendioxybenzyl)-2-oxo-chinolin-3-carbonsäuren, worin T
   1,3-Benzodioxol-5-yl, F. 247-248°
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-6-ethoxy-2-(6-chlor-3,4-methylendioxybenzyloxy)-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-1,2-dihydro-6-ethoxy-1-benzyl-2-oxo-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-6-ethoxy-2-benzyloxy-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-1,2-dihydro-6-ethoxy-1-(2-methylthiobenzyl)-2-oxo-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-6-ethoxy-2-(2-methylthiobenzyloxy)-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-1,2-dihydro-6-ethoxy-1-(2-methylsulfinylbenzyl)-2-oxo-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-6-ethoxy-2-(2-methylsulfinylbenzyloxy)-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-1,2-dihydro-6-ethoxy-1-(2-methylsulfonyl)benzyl-2-oxo-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-6-ethoxy-2-(2-methylsulfonyl)benzyloxy-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-1,2-dihydro-6-ethoxy-1-(2,3-dimethoxy-benzyl)-2-oxo-chinolin-3-carbonsäuren, worin T
   1,3-Benzodioxol-5-yl, F. 211-212°
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-6-ethoxy-2-(2,3-dimethoxy-benzyloxy)-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-1,2-dihydro-6-ethoxy-1-(2,3-methylendioxybenzyl)-2-oxo-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-6-ethoxy-2-(2,3-methylendioxybenzyloxy)-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-1,2-dihydro-6-ethoxy-1-(2,1,3-benzothiadiazol-5-methyl)-2-oxo-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-6-ethoxy-2-(2,1,3-benzothiadiazol-5-methyloxy)-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-1,2-dihydro-1-(2-methoxybenzyl)-2-oxo-chinolin-3-carbonsäuren, worin T
   1,3-Benzodioxol-5-yl, F. 231-232°
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-2-(2-methoxybenzyloxy)-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-1,2-dihydro-1-(3-methoxybenzyl)-2-oxo-chinolin-3-carbonsäuren, worin T
   1,3-Benzodioxol-5-yl, F. 162-163°
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-2-(3-methoxybenzyloxy)-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-1,2-dihydro-1-(4-methoxybenzyl)-2-oxo-chinolin-3-carbonsäuren, worin T
   1,3-Benzodioxol-5-yl, F. 222-223°
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-2-(4-methoxybenzyloxy)-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-1,2-dihydro-1-(3,4-methylendioxybenzyl)-2-oxo-chinolin-3-carbonsäuren, worin T
   1,3-Benzodioxol-5-yl, F. 216-217°
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-2-(3,4-methylendioxybenzyloxy)-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-1,2-dihydro-1-(6-chlor-3,4-methylendioxybenzyl)-2-oxo-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-2-(6-chlor-3,4-methylendioxybenzyloxy)-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-1,2-dihydro-1-benzyl-2-oxo-chinolin-3-carbonsäuren, worin T
   1,3-Benzodioxol-5-yl, F. 184-185°
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-2-benzyloxy-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-1,2-dihydro-1-(2-methylthiobenzyl)-2-oxo-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-2-(2-methylthiobenzyloxy)-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-1,2-dihydro-1-(2-methylsulfinylbenzyl)-2-oxo-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-2-(2-methylsulfinylbenzyloxy)-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-1,2-dihydro-1-(2-methylsulfonylbenzyl)-2-oxo-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-2-(2-methylsulfonylbenzyloxy)-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-1,2-dihydro-1-(2,3-dimethoxy-benzyl)-2-oxo-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-2-(2,3-dimethoxy-benzyloxy)-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-1,2-dihydro-1-(2,3-methylendioxybenzyl)-2-oxo-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-2-(2,3-methylendioxybenzyloxy)-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-1,2-dihydro-1-(2,1,3-benzothiadiazol-5-methyl)-2-oxo-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-2-(2,1,3-benzothiadiazol-5-methylozy)-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-1,2-dihydro-1-(2-methoxybenzyl)-6-propionyl-2-oxo-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-2-(2-methoxybenzyloxy)-6-propionyl-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-1,2-dihydro-1-(3-methoxybenzyl)-6-propionyl-2-oxo-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-2-(3-methoxybenzyloxy)-6-propionyl-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-1,2-dihydro-1-(4-methoxybenzyl)-6-propionyl-2-oxo-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-2-(4-methoxybenzyloxy)-6-propionyl-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-1,2-dihydro-1-(3,4-methylendioxybenzyl)-6-propionyl-2-oxo-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-2-(3,4-methylendioxybenzyloxy)-6-propionyl-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-1,2-dihydro-1-(6-chlor-3,4-methylendioxybenzyl)-6-propionyl-2-oxo-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-2-(6-chlor-3,4-methylendioxybenzyloxy)-6-propionyl-chinolin-3 carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-1,2-dihydro-1-benzyl-6-propionyl-2-oxo-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-2-benzyloxy-6-propionyl-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-1,2-dihydro-6,7-dimethoxy-1-(2-methoxybenzyl)-2-oxo-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-6,7-dimethoxy-2-(2-methoxybenzyloxy)-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-1,2-dihydro-6,7-dimethoxy-1-(3-methoxybenzyl)-2-oxo-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-6,7-dimethoxy-2-(3-methoxybenzyloxy)-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-1,2-dihydro-6,7-dimethoxy-1-(4-methoxybenzyl)-2-oxo-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-6,7-dimethoxy-2-(4-methoxybenzyloxy)-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-1,2-dihydro-6,7-dimethoxy-1-(3,4-methylendioxybenzyl)-2-oxo-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-6,7-dimethoxy-2-(3,4-methylendioxybenzyloxy)-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-1,2-dihydro-6,7-dimethoxy-1-(6-chlor-3,4-methylendioxybenzyl)-2-oxo-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-6,7-dimethoxy-2-(6-chlor-3,4-methylendioxybenzyloxy)-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-1,2-dihydro-6,7-dimethoxy-1-benzyl-2-oxo-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-2-benzyloxy-6,7-dimethoxy-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-1,2-dihydro-6,7-dimethyl-1-(2-methoxybenzyl)-2-oxo-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-6,7-dimethyl-2-(2-methoxybenzyloxy)-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-1,2-dihydro-6,7-dimethyl-1-(3-methoxybenzyl)-2-oxo-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-6,7-dimethyl-2-(3-methoxybenzyloxy)-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-1,2-dihydro-6,7-dimethyl-1-(4-methoxybenzyl)-2-oxo-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-6,7-dimethyl-2-(4-methoxybenzyloxy)-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-1,2-dihydro-6,7-dimethyl-1-(3,4-methylendioxybenzyl)-2-oxo-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-6,7-dimethyl-2-(3,4-methylendioxybenzyloxy)-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-1,2-dihydro-6,7-dimethyl-1-(6-chlor-3,4-methylendioxybenzyl)-2-oxo-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-6,7-dimethyl-2-(6-chlor-3,4-methylendioxybenzyloxy)-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-1,2-dihydro-6,7-dimethyl-1-benzyl-2-oxo-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-2-benzyloxy-6,7-dimethyl-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-1,2-dihydro-6-ethyl-1-(2-methoxybenzyl)-2-oxo-chinolin-3-carbonsäuren, worin T
   1,3-Benzodioxol-5-yl, F. 225-226°
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-6-ethyl-2-(2-methoxybenzyloxy)-chinolin-3-carbonsäuren, worin T
   1,3-Benzodioxol-5-yl
   4-Methoxyphenyl
   2,4-Dimethoxyphenyl.
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-1,2-dihydro-6-ethyl-(3-methoxybenzyl)-2-oxo-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-6-ethyl-2-(3-methoxybenzyloxy)-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-1,2-dihydro-6-ethyl-1-(4-methoxybenzyl)-2-oxo-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-6-ethyl-2-(4-methoxybenzyloxy)-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-1,2-dihydro-6-ethyl-1-(3,4-methylendioxybenzyl)-2-oxo-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-6-ethyl-2-(3,4-methylendioxybenzyloxy)-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-1,2-dihydro-6-ethyl-1-(6-chlor-3,4-methylendioxybenzyl)-2-oxo-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-6-ethyl-2-(6-chlor-3,4-methylendioxybenzyloxy)-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-1,2-dihydro-6-ethyl-1-benzyl-2-oxo-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-6-ethyl-2-benzyloxy-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-1,2-dihydro-6-ethyl-1-(2-methylthiobenzyl)-2-oxo-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-6-ethyl-2-(2-methylthiobenzyloxy)-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-1,2-dihydro-6-ethyl-1-(2-methylsulfinylbenzyl)-2-oxo-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-6-ethyl-2-(2-methylsulfinylbenzyloxy)-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-1,2-dihydro-6-ethyl-1-(2-methylsulfonylbenzyl)-2-oxo-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-6-ethyl-2-(2-methylsulfonylbenzyloxy)-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-1,2-dihydro-6-ethyl-1-(2,3-dimethoxy-benzyl)-2-oxo-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-6-ethyl-2-(2,3-dimethoxy-benzyloxy)-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-1,2-dihydro-6-ethyl-1-(2,3-methylendioxybenzyl)-2-oxo-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-6-ethyl-2-(2,3-methylendioxybenzyloxy)-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-1,2-dihydro-6-ethyl-1-(2,1,3-benzothiadiazol-5-methyl)-2-oxo-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet und
4-T-6-ethyl-2-(2,1,3-benzothiadiazol-5-methyloxy)-chinolin-3-carbonsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet.

### Beispiel 3

Eine Lösung von 4,1 g 4-(1,3-Benzodioxol-5-yl)-2-chlor-chinolin-3-carbonsäuremethylester und 0,25 g Tetrakis(triphenylphosphin)-palladium-(0) in 50 ml Toluol wird mit 10 ml einer 2M wässrigen Natriumcarbonatlösung und 1,52 g 4-Methoxyphenylboronsäure in 25 ml Methanol versetzt und 1 Stunde unter Inertgasatmosphäre unter Rückfluß gekocht. Man arbeitet wie üblich auf und erhält 4-(1,3-Benzodioxol-5-yl)-2-(4-methoxyphenyl)-chinolin-3-carbonsäuremethylester.

### Beispiel 4

Eine Lösung von 3,8 g 4-(1,3-Benzodioxol-5-yl)-2-chlor-chinolin-3-carbonsäuremethylester und 3,1 g Cäsiumcarbonat in 40 ml DMF wird mit einer Lösung von 1,7 g 3,4-Methylendioxyphenol in 20 ml Dichlormethan versetzt und über Nacht gerührt. Man arbeitet wie üblich auf und erhält 4-(1,3-Benzodioxol-5-yl)-2-(3,4-methylendioxy-phenoxy)-chinolin-3-carbonsäuremethylester.

### Beispiel 5

Eine Lösung von 1 g 1,2-Dihydro-1-(2,3-dimethoxybenzyl)-4-(4-methoxyphenyl)-2-oxo-6-nitro-chinolin-3-carbonsäure in 25 ml Methanol wird bei Normaldruck und 20° bis zum Stillstand an 1 g Raney-Nickel hydriert. Man filtriert, entfernt das Lösungsmittel und erhält 6-Amino-1,2-dihydro-1-(2,3-dimethoxybenzyl)-4-(4-methoxyphenyl)-2-oxo-chinolin-3-carbonsäure.

### Beispiel 6

Eine Lösung von 6 g 6-Amino-1,2-dihydro-1-(2,3-dimethoxybenzyl)-4-(4-methoxyphenyl)-2-oxo-chinolin-3-carbonsäure und 0,5 g Titantetrachlorid in 100 ml Methanol wird mit 1 ml frisch destilliertem Acetaldehyd versetzt. Anschließend gibt man 4 g Natriumcyanborhydrid dazu und rührt 30 Stunden. Man gibt halbkonzentrierte Salzsäure dazu, arbeitet wie üblich auf und erhält 6-Ethylamino-1,2-dihydro-1-(2,3-dimethoxybenzyl)-4-(4-methoxyphenyl)-2-oxo-chinolin-3-carbonsäure.

### Beispiel 7

Eine Lösung von 4,85 g 4-(1,3-Benzodioxol-5-yl)-1,2-dihydro-1-(3,4-methylendioxy-benzyl)-2-oxo-chinolin-3-carbonsäure und 0,2 g Dimethylaminopyridin in 50 ml Pyridin wird mit 5 ml Thionylchlorid und 1,57 g Phenylsulfonamid versetzt. Man rührt 10 Stunden, arbeitet wie üblich auf und erhält 4-(1,3-Benzodioxol-5-yl)-1,2-dihydro-1-(3,4-methylendioxy-benzyl)-2-oxo-3-(phenyl-sulfonamidocarbonyl)-chinolin.

### Beispiel 8

Analog Beispiel 1 erhält man durch Umsetzung von 4-(1,3-Benzodioxol-5-yl)-1,2-dihydro-2-oxo-chinolin-3-essigsäuremethylester (erhältlich durch Reaktion von 1-Amino-2-(3,4-methylendioxybenzoyl)-4-ethoxybenzol und Bernsteinsäuremethylesterchlorid) und "A" das N-Alkylierungsprodukt 4-(1,3-Benzodioxol-5-yl)-1,2-dihydro-1-(2-methoxybenzyl)-2-oxo-chinolin-3-essigsäuremethylester, F. 71-72° und das O-Alkylierungsprodukt 4-(1,3-Benzodioxol-5-yl)-2-(2-methoxybenzyloxy)-chinolin-3-essigsäuremethylester.

Analog erhält man aus folgenden 4-T-1,2-dihydro-2-oxo-chinolin-3-essigsäuremethylestern, worin T
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
mit "A" die nachstehenden 4-T-1,2-dihydro-1-(2-methoxybenzyl)-2-oxo-chinolin-3-essigsäuremethylester, worin T
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
sowie die nachstehenden 4-T-2-(2-methoxybenzyloxy)-chinolin-3-essigsäuremethylester, worin T
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet.

Analog erhält man durch Umsetzung von 3,4-Methylendioxybenzylchlorid mit den folgenden 4-T-1,2-dihydro-2-oxo-chinolin-3-essigsäuremethylestern, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
die nachstehenden 4-T-1,2-dihydro-1-(3,4-methylendioxybenzyl)-2-oxo-chinolin-3-essigsäuremethylester, worin T
1,3-Benzodioxol-5-yl, F. 79-80°
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,
sowie die nachstehenden 4-T-2-(3,4-methylendioxybenzyloxy)-chinolin-3-essigsäuremethylester, worin T
1,3-Benzodioxol-5-yl
4-Methoxyphenyl
2,4-Dimethoxyphenyl
2-Benzyloxy-4-methoxyphenyl
2-Hydroxy-4-methoxyphenyl
2-Carboxymethoxy-4-methoxyphenyl
2-Hydroxyethyloxy-4-methoxyphenyl
bedeutet,

Die anschließende Hydrolyse, analog Beispiel 2, der oben aufgeführten Ester ergibt folgende
4-T-1,2-dihydro-1-(2-methoxybenzyl)-2-oxo-chinolin-3-essigsäuren, worin T
   1,3-Benzodioxol-5-yl, F. 165-166°
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bdeutet,
4-T-2-(2-methoxybenzyloxy)-chinolin-3-essigsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
4-T-1,2-dihydro-1-(3,4-methylendioxybenzyl)-2-oxo-chinolin-3-essigsäuren, worin T
   1,3-Benzodioxol-5-yl, F. 186-187°
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet,
und 4-T-2-(3,4-methylendioxybenzyloxy)-chinolin-3-essigsäuren, worin T
   1,3-Benzodioxol-5-yl
   4-Methoxyphenyl
   2,4-Dimethoxyphenyl
   2-Benzyloxy-4-methoxyphenyl
   2-Hydroxy-4-methoxyphenyl
   2-Carboxymethoxy-4-methoxyphenyl
   2-Hydroxyethyloxy-4-methoxyphenyl
bedeutet.

### Beispiel 9

Analog Beispiel 2 erhält man durch Hydrolyse
aus 4-(1,3-Benzodioxol-5-yl)-1,2-dihydro-5,6-dimethoxy-1-(2-methoxybenzyl)-2-oxo-chinolin-3-carbonsäuremethylester, F. 210-211°
   4-(1,3-Benzodioxol-5-yl)-1,2-dihydro-5,6-dimethoxy-1-(2-methoxybenzyl)-2-oxo-chinolin-3-carbonsäure` F. 250°
aus 4-(1,3-Benzodioxol-5-yl)-1,2-dihydro-5,6-dimethoxy-1-(2-methylbenzyl)-2-oxo-chinolin-3-carbonsäuremethylester
   4-(1,3-Benzodioxol-5-yl)-1,2-dihydro-5,6-dimethoxy-1-(2-methylbenzyl)-2-oxo-chinolin-3-carbonsäure` F. 247-249°
aus 4-(1,3-Benzodioxol-5-yl)-1,2-dihydro-5,6-dimethoxy-1-(2,5-dimethoxybenzyl)-2-oxo-chinolin-3-carbonsäuremethylester
   4-(1,3-Benzodioxol-5-yl)-1,2-dihydro-5,6-dimethoxy-1-(2,5-dimethoxybenzyl)-2-oxo-chinolin-3-carbonsäure, F. 233°
aus 4-(1,3-Benzodioxol-5-yl)-1,2-dihydro-5,6-dimethoxy-1-(3,4,5-trimethoxybenzyl)-2-oxo-chinolin-3-carbonsäuremethylester
   4-(1,3-Benzodioxol-5-yl)-1,2-dihydro-5,6-dimethoxy-1-(3,4,5-trimethoxybenzyl)-2-oxo-chinolin-3-carbonsäure. F. 269,3°
aus 4-(4-Methoxyphenyl)-1,2-dihydro-6-ethoxy-1-(2,5-dimethoxybenzyl)-2-oxo-chinolin-3-carbonsäuremethylester, F. 198-199°
   4-(4-Methoxyphenyl)-1,2-dihydro-6-ethoxy-1-(2,5-dimethoxybenzyl)-2-oxo-chinolin-3-carbonsäure, F. 178-179°
aus 4-(1,3-Benzodioxol-5-yl)-1,2-dihydro-6-ethyl-1-(2,5-dimethoxybenzyl)-2-oxo-chinolin-3-carbonsäuremethylester
   4-(1,3-Benzodioxol-5-yl)-1,2-dihydro-6-ethyl-1-(2,5-dimethoxybenzyl)-2-oxo-chinolin-3-carbonsäure, F. 199,2°.

Die nachfolgenden Beispiele betreffen pharmazeutische Zubereitungen:

### Beispiel A: Injektionsgläser

Eine Lösung von 100 g eines Wirkstoffes der Formel I und 5 g Dinatriumhydrogenphosphat wird in 3 l zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I, 9,38 g NaH₂PO₄ · 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 l auf und sterilisiert durch Bestrahlung. Diese Lösung kann in Form von Augentropfen verwendet werden.

### Beispiel D: Salbe

Man mischt 500 mg eines Wirkstoffes der Formel I mit 99,5 g Vaseline unter aseptischen Bedingungen.

### Beispiel E: Tabletten

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 l zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Verbindungen der Formel I 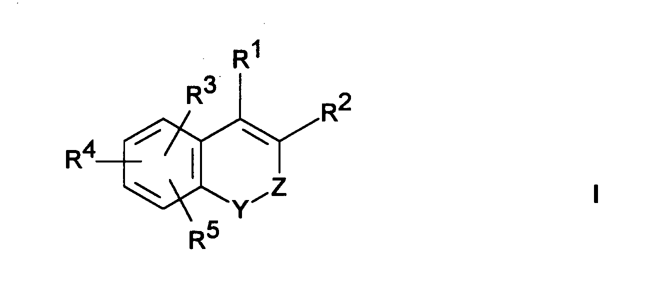 worin
-Y-Z- -NR⁷-CO-, -N=C(OR⁷)- oder -N=CR⁸-,
R¹ Ar,
R² COOR⁶, (CH₂)ₙCOOR⁶, CN, 1H-Tetrazol-5-yl oder CONHSO₂Ar,
R³, R⁴, R⁵ jeweils unabhängig voneinander R⁶, OR⁶, S(O)ₘR⁶, Hal, NO₂, NR⁶R⁶', NHCOR⁶, NHSO₂R⁶, OCOR⁶, COR⁶, COOR⁶ oder CN, wobei R³ und R⁴ zusammen auch eine O(CH₂)ₙO-Gruppe darstellen können,
R⁶, R^{6'} jeweils unabhängig voneinander H, Alkyl mit 1 bis 6 C-Atomen, Benzyl oder Phenyl,
R⁷ (CH₂)ₙAr,
R⁸ Ar oder OAr,
Ar unsubstituiertes oder ein-, zwei- oder dreifach durch R⁹, R¹⁰ oder R¹¹ substituiertes Phenyl oder
unsubstituiertes Naphthyl oder
eine unsubstituierte oder im Phenylteil ein- oder zweifach durch R⁹ oder R¹⁰ substituierte 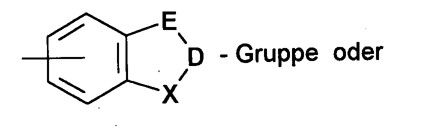 eine unsubstituierte oder im Cyclohexadienylteil ein- oder zweifach durch R⁹ oder R¹⁰ substituierte 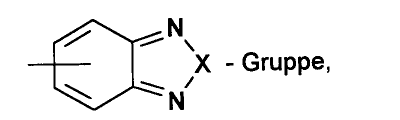
R⁹, R¹⁰, R¹¹ jeweils unabhängig voneinander R⁶, OR⁶, Hal, CF₃, OCF₃, OCHF₂, OCH₂F, NO₂, NR⁶R^{6'}, NHCOR⁶, CN, NHSO₂R⁶, COOR⁶, COR⁶, CONHSO₂Ar, O(CH₂)ₙR², O(CH₂)ₙOR⁶ oder S(O)ₘR⁶,
E CH₂, S oder O,
D Carbonyl oder [C(R⁶R^{6'})]ₙ,
X O oder S,
Hal F, Cl, Br oder I,
m 0, 1 oder 2,
n 1, 2 oder 3 bedeuten,
sowie ihre Salze.

2. Verbindungen der Formel I gemäß Anspruch 1
a) 4-(1,3-Benzodioxol-5-yl)-1,2-dihydro-1-(2-methoxybenzyl)-2-oxochinolin-3-carbonsäure;
b) 4-(1,3-Benzodioxol-5-yl)-1,2-dihydro-1-(4-methoxybenzyl)-2-oxochinolin-3-carbonsäure;
c) 4-(1,3-Benzodioxol-5-yl)-1,2-dihydro-1-(3,4-methylendioxybenzyl)-2-oxochinolin-3-carbonsäure;
d) 4-(1,3-Benzodioxol-5-yl)-1,2-dihydro-1-(2-methoxybenzyl)-2-oxochinolin-3-essigsäure;
e) 4-(1,3-Benzodioxol-5-yl)-1,2-dihydro-1-(3,4-methylendioxybenzyl)-2-oxochinolin-3-essigsäure;
f) 4-(1,3-Benzodioxol-5-yl)-1,2-dihydro-6-ethoxy-1-(2-methoxybenzyl)-2-oxochinolin-3-carbonsäure;
g) 4-(1,3-Benzodioxol-5-yl)-1,2-dihydro-6-ethoxy-1-(4-methoxybenzyl)-2-oxochinolin-3-carbonsäure;
h) 4-(1,3-Benzodioxol-5-yl)-1,2-dihydro-6-ethoxy-1-(6-chlor-3,4-methylendioxybenzyl)-2-oxochinolin-3-carbonsäure;
i) 4-(1,3-Benzodioxol-5-yl)-1,2-dihydro-6-ethoxy-1-(3,4-methylendioxybenzyl)-2-oxochinolin-3-carbonsäure;
j) 4-(1,3-Benzodioxol-5-yl)-1,2-dihydro-6-ethoxy-1-(3-methoxybenzyl)-2-oxochinolin-3-carbonsäure;

3. Verfahren zur Herstellung von Verbindungen der Formel I nach Anspruch 1 sowie ihrer Salze, dadurch gekennzeichnet daß man
(a) zur Herstellung einer Verbindung der Formel I
worin
-Y-Z- -NR⁷-CO- oder -N=C(OR⁷)- bedeutet,
eine Verbindung der Formel II 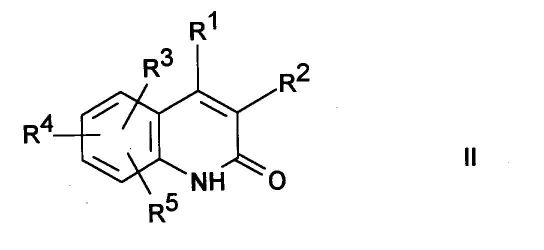 worin
R¹, R², R³, R⁴ und R⁵ die in Anspruch 1 angegebenen Bedeutungen haben,
mit einer Verbindung der Formel III
R⁷-Q III
worin
Q Cl, Br, I oder eine freie oder reaktionsfähig funktionell abgewandelte OH-Gruppe bedeutet und
R⁷ die in Anspruch 1 angegebene Bedeutung hat,
umsetzt,
oder daß man
(b) zur Herstellung einer Verbindung der Formel I,
worin
-Y-Z- -N=C(Ar)- bedeutet,
eine Verbindung der Formel IV 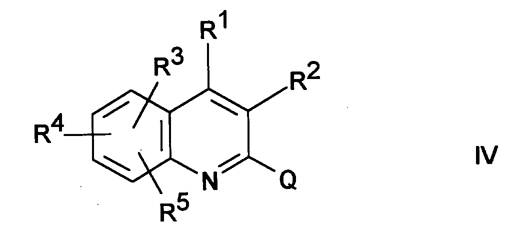 worin
Q Cl, Br oder I bedeutet und
R¹, R², R³, R⁴ und R⁵ die in Anspruch 1 angegebenen Bedeutungen haben,
mit einer Borverbindung der Formel V
Ar-BLL' V
worin
L, L' jeweils unabhängig voneinander OH, OCH₃, OC₂H₅ oder OC₃H₇ bedeutet, und
Ar die in Anspruch 1 angegebene Bedeutung hat, umsetzt,
oder daß man
c) zur Herstellung einer Verbindung der Formel I,
worin
-Y-Z- -N=C(OAr)- bedeutet,
eine Verbindung der Formel IV
worin
Q Cl, Br, I oder eine reaktionsfähig funktionell abgewandelte OH-Gruppe bedeutet,
mit einer Verbindung der Formel VI
Ar-OH VI
umsetzt,
und/oder daß man in einer Verbindung der Formel I einen oder mehrere Rest(e) R¹, R², R³, R⁴ und/oder R⁵ in einen oder mehrere Rest(e) R¹, R², R³, R⁴ und/oder R⁵ umwandelt,
indem man beispielsweise
i) eine Nitrogruppe zu einer Aminogruppe reduziert,
ii) eine Estergruppe zu einer Carboxygruppe hydrolysiert,
iii) eine Aminogruppe durch reduktive Aminierung in ein alkyliertes Amin umwandelt,
iv) eine Carboxygruppe in eine Sulfonamidocarbonylgruppe umwandelt
und/oder eine Base oder Säure der Formel I in eines ihrer Salze umwandelt.

4. Verfahren zur Herstellung pharmazeutischer Zubereitungen, dadurch gekennzeichnet, daß man eine Verbindung der Formel I nach Anspruch 1 und/oder eines ihrer physiologischen unbedenklichen Salze zusammen mit mindestens einem festen, flüssigen oder halbflüssigen Träger- oder Hilfsstoff in eine geeignete Dosierungsform bringt.

5. Pharmazeutische Zubereitung, gekennzeichnet durch einen Gehalt an mindestens einer Verbindung der Formel I nach Anspruch 1 und/oder einem ihrer physiologisch unbedenklichen Salze.

6. Verbindungen der Formel I nach Anspruch 1 und ihre physiologisch unbedenklichen Salze zur Bekämpfung von Krankheiten.

7. Arzneimittel der Formel I nach Anspruch 1 und ihre physiologisch unbedenklichen Salze als Endothelin-Rezeptor-Antagonisten.

8. Verwendung von Verbindungen der Formel I nach Anspruch 1 und/oder ihre physiologisch unbedenklichen Salze zur Herstellung eines Arzneimittels.

9. Verwendung von Verbindungen der Formel I nach Anspruch 1 und/oder ihrer physiologisch unbedenklichen Salze bei der Bekämpfung von Krankheiten.
